# EUROPEAN PATENT APPLICATION

(11) **EP 4 674 971 A1**
(43) Date of publication of application: **07.01.2026**
(21) Application number: 24186115.2
(22) Date of filing: 02.07.2024
(51) Int. Cl.: C12P 7/26, C07K 14/47, C12N 9/10, C12N 15/52, C12P 7/6409, C12P 17/08

(54) **METHODS FOR ACYL GROUP TRANSFER AND POLYKETIDE SYNTHESIS**

(71) Applicant: kez.biosolutions GmbH, 14476 Potsdam (DE)
(72) Inventor: Joppe, Mirko, 14476 Potsdam-Golm (DE); Rittner, Alexander, 14476 Potsdam-Golm (DE); Degen, Jan, 14476 Potsdam-Golm (DE)
(74) Representative: Eisenführ Speiser

(57) **Abstract**

The present invention relates to the fields of fatty acid- and polyketide synthesis, and protein engineering. In particular, it relates to methods of using altered acyltransferases having an altered coenzyme specificity for acyl group transfer and polyketide synthesis.

## Description

### Technical field

The present invention relates to the fields of fatty acid- and polyketide synthesis, and protein engineering. In particular, it relates to methods of using altered acyltransferases having an altered coenzyme specificity for acyl group transfer and polyketide synthesis.

### Background

Polyketides represent a versatile and important class of biomolecules. Particularly polyketide antibiotics, immunosuppressants, antiparasitics, as well as antifungal, cholesterol-lowering, and antitumoral agents represent important classes of biomolecules of outstanding therapeutic interest.

Despite having a biomolecular target, most polyketides are currently not used as therapeutics so that only a tiny fraction of their great potential is used for this particular substance class. In order to make them applicable for the human body, polyketides need to be tailored with certain functionalities. However, organic synthesis is very challenging and economically unfeasible for this particular substance class, due to their large carbon scaffolds with many functional groups and stereochemical properties. There is thus a great need in the pharmaceutical and biotechnological industry to provide systems allowing the synthesis of new and particularly custom-made polyketides with tailored functionalities in a reasonable time.

While polyketide synthase (PKS) systems bear a vast potential for synthetizing myriads of different biologically active compounds for drug design and other application, PKS and fatty acid synthase (FAS) enzymes are limited to substrates bound to Coenzyme A (CoA). CoA is composed of cysteamine (β-mercaptoethylamine), β-alanine, pantoic acid, organophosphate anhydride and a 3'-phosphoadenosine. The thiol group of the cysteamine can form a "high energy" thioester bond with carboxylic acids thereby forming "activated" acyl-CoA molecules.

CoA, however, is an abundant molecule in typical production strains so that naturally present CoA-bound substrates can interfere with polyketide synthesis. Consequently, having enzymes available with a decreased or even abolished specificity for CoA could be advantageous for the *in vivo* production of polyketides during metabolic engineering to shift the product yield towards a desired ketide or polyketide compound of interest using specifically designed enzymes as engineered molecular machines. In addition, using an artificial pathway (relying on acyl substrates bound to a different coenzyme moiety) for the biosynthesis of a target compound, avoids interference with the natural metabolism and a new functionality does no longer require identifying and establishing a suitable natural metabolic pathway to produce the substrate. It was thus an object of the present invention to provide methods of acyl group transfer as well as polyketide synthesis that overcome these problems associated with CoA-bound substrates and to expand the currently available production methods.

Crystal structures of some FAS enzymes and PKS systems have been solved (Smith and Tsai 2007, Rittner *et al.* 2020). However, it has (to our knowledge) never been envisioned to use acyltransferases with an altered CoA binding pocket that, ideally, does not use acyl-CoA substrates and accepts subunits bound to different (non-CoA) coenzyme moieties, such as synthetic coenzyme moieties.

It was a particular object to expand and re-define the substrate recognition, binding and catalysis spectrum of acyltransferases to provide new methods allowing the use of a wide range of substrates, ideally while excluding CoA, to allow for targeted polyketide(-precursor) synthesis, preferably the synthesis of ketides or polyketide building blocks comprising positions within the polyketide molecule, where synthetic moieties can be inserted or added already during synthesis *in vivo* when produced by a host cell of interest or *in vitro* when produced in an artificial enzymatic system, or by semi-synthetic processes after production of the (poly-)ketide scaffold.

### Summary of the Invention

In a first aspect, there is provided a method comprising: (a) providing (i) at least one non-CoA acyltransferase substrate; and (ii) at least one altered acyltransferase ; and (iii) at least one acyl carrier protein (ACP); (b) allowing the at least one acyltransferase to transfer the acyl group of the at least one non-CoA acyltransferase substrate to the at least one ACP; (c) obtaining at least one acyl-ACP; wherein the at least one altered acyltransferase has a CoA-binding pocket that is altered to reduce CoA acyltransferase substrate transfer activity.

In one embodiment of the first aspect, step (b) is performed in at least one cell comprising the components (i), (ii), and (iii) of step (a).

In one embodiment of the first aspect, step (b) is performed outside of a cell.

In one embodiment of the first aspect, step (b) the at least one altered acyltransferase is provided in a cell-free system and/or as a purified or partially purified protein or as a lysate comprising the altered acyltransferase, optionally wherein the at least one altered acyltransferase is provided as a fusion protein further comprising at least one linker domain and optionally at least one ketoacyl synthase domain.

In a second aspect, there is provided a method for polyketide synthesis, the method comprising: (a) providing (i) at least one non-CoA acyltransferase substrate; and (ii) optionally at least one CoA acyltransferase substrate; and (ii) a polyketide synthase (PKS) system, wherein the PKS system comprises at least one altered acyltransferase; (b) allowing polyketide synthesis, wherein the at least one non-CoA acyltransferase substrate, and optionally the at least one CoA acyltransferase substrate, is/are used as a substrate in the polyketide synthesis; (c) obtaining at least one polyketide, and optionally purifying said at least one polyketide; wherein the at least one altered acyltransferase has a CoA-binding pocket that is altered to reduce CoA acyltransferase substrate transfer activity.

In one embodiment of the second aspect, the PKS system is a PKS type I system, wherein one, two, three or more PKS modules comprise and/or interact with at least one altered acyltransferase.

In one embodiment of the second aspect, the PKS system is a PKS type II system, wherein the individual catalytic proteins of the PKS system interact with at least one altered acyltransferase.

In one embodiment of the second aspect, the first, second, third, fourth, fifth, six, seventh, eighth, ninth, tenth, eleventh, thirteenth, fourteenths, fifteenth, and/or sixteenth PKS module and/or one or more subsequent PKS modules comprise(s) and/or interact(s) with at least one altered acyltransferase.

In one embodiment of the second aspect, the at least one altered acyltransferase is part of at least one multifunctional PKS type I polypeptide, optionally through replacement of at least one of the PKS acyltransferases.

In one embodiment of the second aspect, the at least one altered acyltransferase is provided as a separate polypeptide, optionally as a fusion protein further comprising at least one linker domain and optionally at least one ketoacyl synthase domain.

In one embodiment of the second aspect, the polyketide synthesis, or part of the polyketide synthesis, is performed in at least one cell comprising the PKS system or parts thereof.

In one embodiment of the second aspect, the polyketide synthesis, or part of the polyketide synthesis, is performed outside of a cell.

In one embodiment of the second aspect, the at least one altered acyltransferase is provided in a cell-free system and/or as a purified or partially purified protein or as a lysate comprising the altered acyltransferase, optionally wherein the at least one altered acyltransferase is provided as a fusion protein further comprising at least one linker domain and optionally at least one ketoacyl synthase domain.

In one embodiment of the first or second aspect, wherein the at least one altered acyltransferase has a CoA-binding pocket that is altered to reduce CoA acyltransferase substrate transfer activity and optionally to increase transfer activity for the at least one non-CoA acyltransferase substrate.

In one embodiment of the first or second aspect, the at least one altered acyltransferase has a CoA-binding pocket that is altered to transfer the at least one non-CoA acyltransferase substrate at a higher rate than the equivalent CoA acyltransferase substrate with the same acyl group and/or, in case at least one CoA acyltransferase substrate is provided in step (a), the at least one CoA acyltransferase substrate provided in step (a).

In one embodiment of the first or second aspect, the at least one non-CoA substrate comprises or consists of (ii) a non-CoA coenzyme; and (i) an acyl group; optionally wherein part (i) and part (ii) are linked via a thioester.

In one embodiment of the first or second aspect, the non-CoA coenzyme has a neutral net charge or a positive net charge.

In one embodiment of the first or second aspect, the positive net charge is a single, two-fold, three-fold or four-fold positive net charge.

In one embodiment of the first or second aspect, the non-CoA coenzyme does not comprise a negative charge.

In one embodiment of the first or second aspect, the non-CoA coenzyme does not comprise a negative charge that is within 10 to 25 angstrom of the high energy bond, preferably the ester bond, more preferably the thioester bond, linking the non-CoA coenzyme to the acyl group.

In one embodiment of the first or second aspect, the acyl group comprises or consist of a malonyl group, an acetyl group, a methylmalonyl group, a propionyl group, or a derivative thereof.

In a third aspect, there is provided a use of at least altered acyltransferase as defined in the first or second aspect, wherein the at least one altered acyltransferase originates from a PKS; and/or at least one non-CoA acyltransferase substrate as defined in the second aspect, and optionally at least one CoA acyltransferase substrate for polyketide synthesis, fatty acid synthesis, and/or nonribosomal peptide synthesis.

In a fourth aspect, there is provided a kit comprising at least one altered acyltransferase as defined in the first or second aspect; and/or at least one non-CoA acyltransferase substrate as defined in the second aspect; optionally further comprising at least one CoA acyltransferase substrate, and/or at least one container, and/or a set of reagents including buffer and/or medium, and/or means of transfection. A kit will usually comprise all agents, buffers, co-factors etc. necessary to guarantee the functionality of the at least one acyltransferase in an assay of interest.

### Definitions

An "acyltransferase" or "AT" as used herein refers to a fatty acid synthase (FAS) acyltransferase or polyketide synthase (PKS) acyltransferase having an Enzyme Commission number selected from EC 2.3.1.39 ([acyl-carrier-protein] S-malonyltransferase) or EC 2.3.1.38 ([acyl-carrier-protein] S-acetyltransferase). An acyltransferase can selectively bind and trans-esterify different coenzyme-bound acyl molecules onto an acyl carrier protein (ACP), for instance as part of fatty acid or polyketide synthesis. While natural acyltransferases are specific for coenzyme A (CoA)-bound acyl molecules ("CoA acyltransferase substrates"), an acyltransferase of the present invention is an artificially altered acyltransferase that no longer accepts acyl-CoA substrates and can instead bind and trans-esterify acyl groups bound to other coenzyme moieties, including synthetic coenzyme moieties ("non-CoA acyltransferase substrates"). An acyltransferase of the present invention may be expressed as part of a FAS and/or PKS, or a sub-part thereof, as a separate molecule and/or as part of a fusion protein. An "altered acyltransferase" as used herein, refers to an acyltransferase having an altered CoA-binding pocket according to the present invention leading to a reduced CoA acyltransferase substrate transfer activity.

An acyltransferase "originating from a FAS" refers to an acyltransferase for which the corresponding, wild type acyltransferase (AT) is part of said fatty acid synthase in nature, wherein an acyltransferase of the present invention may be present as part of said fatty acid synthase or may be an isolated acyltransferase according to the present disclosure. For instance, for the altered acyltransferase according to SEQ ID NO: 57 the corresponding wild type malonyl-acetyl-transferase (MAT) sequence is SEQ ID NO: 29, which is part of the fatty acid synthase according to SEQ ID NO: 1; hence the altered acyltransferase according to SEQ ID NO: 57 originates from the fatty acid sequence according to SEQ ID NO: 1. Various wild type ATs and the fatty acid synthases they are part of in nature, are disclosed as SEQ ID NOs: 29 to 56 and SEQ ID NOs: 1 to 28, respectively. An acyltransferase "originating from a PKS" refers to an acyltransferase for which the corresponding, wild type acyltransferase is part of said PKS in nature, wherein an acyltransferase of the present invention may be present as part of said PKS or may be an isolated acyltransferase according to the present disclosure. For instance, for the altered acyltransferase according to SEQ ID NO: 154 the corresponding wild type AT sequence is SEQ ID NO: 139, which is part of module 6 of the 6-deoxyerythronolide-B synthase EryA3, a PKS in *Saccharopolyspora erythraea*; hence the altered acyltransferase according to SEQ ID NO: 154 originates from the 6-deoxyerythronolide-B synthase EryA3. The skilled person can easily determine, for instance through sequence and/or structural alignments, the acyltransferase within a given FAS or PKS sequence. An acyltransferase may also be any fragment, part and/or (sub)domain of a FAS or PKS acyltransferase, as long as it has acyltransferase activity.

An "isolated acyltransferase" as used herein, refers to an altered acyltransferase that is not present as part of a continuous polypeptide comprising the complete FAS or PKS from which it originates, optionally not comprising more than the acyltransferase and optionally the linker domain (LD), or a part thereof, and/or the ketoacyl synthase (KS) of the FAS or PKS from which it originates. An isolated acyltransferase may be present as a discrete enzyme, i.e. a polypeptide consisting of said acyltransferase. Preferably, a polypeptide comprising an isolated acyltransferase further comprises at least one protein, domain or part thereof to increase the stability, such as an LD, or a part thereof, wherein the LD may originate from the same FAS or PKS or may originate, in form of a fusion protein, from at least one different FAS and/or PKS, or a different protein, domain or part thereof, for example an MBP-tag or a different tag. In certain embodiments, an isolated acyltransferase is present as part of a polypeptide comprising the acyltransferase, a KS as well as a LD connecting the acyltransferase and the KS, wherein the KS, the LD may originate from the same FAS or PKS or may originate, in form of a fusion protein, from at least one different FAS and/or PKS. Generally, an isolated acyltransferase may be present as part of a fusion protein further comprising at least one polypeptide that does not originate from the same FAS or PKS or does not originate from any FAS or PKS, such as one or more different enzyme parts and/or domains, including FAS and/or PKS functional domains, one or more tags, one or more binding domains, synthetic zippers and/or the like.

An "amino acid exchange" as used herein, refers to the presence of an amino acid at a given position of the primary amino acid sequence of a polypeptide that differs from the amino acid present at said position of the primary amino acid sequence of a reference polypeptide, such as a wild type polypeptide. Commonly, amino acid exchanges are achieved by mutating the respective codon of a nucleic acid sequence encoding the polypeptide into a codon encoding a different amino acid at said position. Means and methods to exchange an amino acid at a desired position, for instance but not limited to PCR-based cloning methods as disclosed herein, are well established in the field and available to the skilled person.

The terms "CoA-binding pocket" and "CoA-binding region" are used interchangeably herein and refers to the region of the acyltransferase that binds (when not altered according to the present invention) to the CoA moiety for and/or during the acyl group transfer and that comprises one or more or all of the reference positions according the present invention. An altered CoA-binding pocket according to the present invention is a CoA binding pocket that has been altered to reduce the CoA acyltransferase substrate transfer activity and optionally to increase the substrate transfer activity for at least one desired non-CoA acyltransferase substrate. An altered CoA binding pocket may be achieved by one or more amino acid exchanges within the CoA-binding pocket and/or by one or more amino acid exchanges outside of the CoA-binding pocket altering the structure of the CoA-binding pocket. The skilled person can use the natural CoA acyltransferase substrate(s) in methods known in the art and/or described herein measuring the acyl group transfer and/or the release of free CoA directly or indirectly in order to verify a reduced CoA acyltransferase substrate transfer activity. For polyspecific acyltransferases, the skilled person can use one of the natural CoA acyltransferase substrates as an exemplary CoA acyltransferase substrate to determine whether the CoA acyltransferase substrate transfer activity is reduced. In embodiments specifying a percentage of reduced CoA acyltransferase substrate transfer activity, it is sufficient for polyspecific acyltransferases that the transfer activity of one of the natural CoA acyltransferase substrate is reduced to the specified percentage range. A "natural CoA acyltransferase substrate" is the substrate or one of the substrates of a given acyltransferase without the altered CoA-binding pocket according to the present invention. Altering of the CoA-binding pocket according to the present invention may be used in combination with one or more further mutations changing the acyl specificity of the acyltransferase (in addition to the altered coenzyme specificity according to the present invention). In this case, the natural CoA acyltransferase substrate(s) is/are the CoA acyltransferase substrate(s) of that mutated acyltransferase with a changed acyl specificity but without the altered CoA-binding pocket.

A reduced CoA acyltransferase substrate transfer activity according to the present invention describes a change in the coenzyme-binding properties due to the altered CoA-binding region, thereby leading to an altered coenzyme specificity. A reduced CoA acyltransferase substrate transfer activity according to the present invention does not include a reduction of substrate transfer activity due to a change in acyl specificity or an overall inactivation of the AT, for example due to denaturing. However, in certain embodiments, the at least one altered acyltransferase may have an altered acyl specificity in addition to the reduced CoA acyltransferase substrate transfer activity. A non-CoA acyltransferase substrate may be used to verify the non-CoA acyltransferase substrate activity (for instance *S*-Malonyl-*N*-hexanoylcysteamine as shown in example 4). In preferred embodiments, an acyltransferase having an altered CoA-binding pocket is an acyltransferase having an amino acid exchange to reduce the CoA acyltransferase substrate transfer activity at 1, 2, 3, 4, 5, 6, 7 or 8 reference position(s) according to table 1 to table 5.

A "fatty acid synthase" or "FAS" as used herein, refers to a fatty acid synthase of type I, being a multi-functional protein, also called a multi-enzyme, capable of catalyzing all steps of fatty acid synthesis or a fatty acid synthase of type II comprising multiple monofunctional enzymes for fatty acid synthesis. In nature, wild type fatty acid synthases catalyze multiple rounds of Claisen condensation reactions with acetyl-CoA and malonyl-CoA recognized and transferred by an acyltransferase being a malonyl-acetyl-transferase (MAT). The large multi-functional proteins comprise the functional domains of an acyltransferase transferring the acyl-substrates onto an acyl carrier protein (ACP), a ketoacyl synthase (KS) catalyzing the Claisen condensation reaction, a ketoacyl reductase (KR), a hydroxyacyl dehydratase (DH) and an enoyl reductase (ER) creating a fully saturated acyl backbone and a thioesterase (TE) releasing the synthesized fatty acid. (Smith and Tsai 2007).

A "fusion protein" as used herein refers to a polypeptide that is created by joining at least two different polypeptides, which may each be a complete protein existing in nature, a part thereof, or a fully synthetic polypeptide, to form one continuous artificial polypeptide. Commonly, a fusion protein is produced by fusing the nucleic acid sequences of the polypeptides to be joined. Means and methods to clone and express fusion proteins are well-established in the field and available to the skilled person. Fusion proteins of the present disclosure may for instance comprise one or more tags, such as affinity tags, including at least one His-tag, strep-tag, SNAP-tag, HA-tag and the like, or combinations thereof, e.g., an N-terminal His-tag and a C-terminally located strep-tag, or vice versa, epitope tags, fluorescence tags, solubilization tags, synthetic zippers or the like. A cleavable tag that can be removed after production may be used as well. Usually, tags will be located at the N- or C-terminus of a polypeptide not to disturb the function of the folded protein, but in certain cases, a tag may also be located within a polypeptide sequence (e.g., forming a discrete structural epitope not disturbing the fold and function of the polypeptide the tag is inserted in). Further, one or more linker sequences may be used, including a FAS and/or PKS LD, and/or other linkers (including synthetic linkers) or spacers or flexible linkers to connect the different entities of a fusion molecule with each other so that the different entities can exert there function appropriately (e.g., "GA" linkers or spacers of various length building alpha-helical structures may be used, one or more binding or docking domains, one or more dimerization domains, such as leucine zippers).

Whenever the present disclosure relates to the percentage of the identity of nucleic acid or amino acid sequences, this identity is determined by a comparison of a sequence of interest, the reference sequence (e.g., a SEQ ID NO as disclosed herein), to another sequence, the query sequence, over the entire length of the reference sequence. Identity is obtained by using the EMBOSS Water Pairwise Sequence Alignments (nucleotide) program or the EMBOSS Water Pairwise Sequence Alignments (protein) program (www.ebi.ac.uk/Tools/psa/emboss_water/) for amino acid sequences. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5.

A "linker domain" or "LD" as used herein refers to both linker sequences upstream and downstream of an acyltransferase within a FAS or PKS that - together - link the acyltransferase to the KS, both sub-parts also being referred to as KS-AT (or KS-MAT) Linker and post-AT Linker. A linker domain as disclosed herein may comprise or consist of both sub-parts from the same FAS or PKS or a linker domain may be hybrid comprising or consisting of a KS-(M)AT Linker and a post-AT Linker, each stemming from a different FAS or PKS.

A "negatively charged" amino acid as used herein, refers to aspartate, glutamate, histidine or a non-natural amino acid or amino acid analog carrying a negative net charge at least at certain pH conditions. The terms "aspartate" and "glutamate" are used interchangeably with the terms "aspartic acid" and "glutamic acid", respectively, herein, irrespective of the protonation state. Notably, histidine can be negatively or positively charged depending on the pH. Further, a neutral charge already at pH ~ 8.0 is possible. Therefore, depending on the pH and in view of the pl, histidine can be qualified as negatively charged residue, but also differently.

A "reference position" as used herein, refers to one of the conserved positions (i), (ii), (iii), (iv), (v), (vi), (vii) or (viii) as defined in Tables 1 to Table 5. For an acyltransferase originating, for example, from a murine FAS according to SEQ ID NO: 1, the reference positions (i) to (viii) correspond to the positions D160, T161, F184, R286, K186, K285, R300, A282, and G137 with the numbering based on the sequence of the acyltransferase domain itself (SEQ ID NO: 29), which corresponds to positions D647, T648, F671, R773, K673, K772, R787, A769, and G624 with the numbering based on the sequence of the complete murine FAS (SEQ ID NO: 1). For an acyltransferase originating from a chicken FAS according to SEQ ID NO: 14, as a further example, the reference positions (i) to (viii) correspond to the positions D160, T161, F184, R286, K186, R285, K300, A282, and G137 with the numbering based on the sequence of the acyltransferase domain itself (SEQ ID NO: 42), which corresponds to positions D646, T647, F670, R772, K672, R771, K786, A768 and G623 with the numbering based on the sequence of the complete chicken FAS (SEQ ID NO: 14). When comparing different sequences of acyltransferases according to the present disclosure (e.g. SEQ ID NOs: 29 to 56 or SEQ ID NOs: 134 to 153) numbers of positions corresponding to the same reference position may be identical (when the numbering is based on the acyltransferase itself) or may differ.

The reference positions disclosed herein are not limited to the sequences of SEQ ID NOs: 1 to 28 or SEQ ID NOs: 29 to 56 or SEQ ID NOs: 134 to 153. Sequence alignments, such as shown in Table 1 to Table 5, are well-established in the field and the skilled person can easily determine for any given acyltransferase sequence the positions of reference positions (i), (ii), (iii), (iv), (v), (vi), (vii) and/or (viii) within that sequence and further determine whether one or more of the reference positions are present in that sequence. As the structure of the protein fold is well conserved, the skilled person may additionally use structural alignments, for instance based on x-ray cristallography, (cryo-)EM and/or alphafold structures, in combination with sequence alignments in certain cases, such as for sequences that are less well conserved, to determine the reference positions. Those may, for instance, be a threonine, serine, glutamine or asparagine residue at reference position (i), a phenylalanine, arginine, lysine, glutamate or histidine residue at reference position (ii), an arginine, glutamate, aspartate or histidine residue at reference position (iii), a lysine, glutamine or arginine residue at reference position (iv), a lysine or arginine residue at reference position (v), a lysine or arginine residue at reference position (vi), an alanine, serine, valine or glycine residue at reference position (vii) and/or a glycine, alanine, proline or serine residue at reference position (viii).

Whenever the present disclosure refers to an "analogous" residue in the context to SEQ ID NO: 29, it refers to the residue present in a different sequence at the position that corresponds to the same reference position. For example, T161 of SEQ ID NO: 14 is an analogous threonine residue to T161 of SEQ ID NO: 29 and R285 of SEQ ID NO 14 is an analogous arginine residue to K285 of SEQ ID NO: 29. While the numbering based on SEQ ID NO: 14 is identical to the numbering based on SEQ ID NO: 29, an analogous residue may not necessarily have the identical number as the respective residue in SEQ ID NO: 29, depending on the individual sequence.

A "polyketide synthase" or "PKS" as used herein, refers to a type I PKS, being a large multifunctional protein that has a modular enzymatic structure, in which one module comprises a set of functional enzymatic domains involved in the incorporation of an α-carboxyacyl extender unit into a growing polyketide chain, or a type II PKS comprising discrete monofunctional proteins for polyketide synthesis. A type I PKS module comprises at least three different functional domains: a ketoacyl synthase (KS), which is responsible for catalyzing the Claisen condensation between an extender unit and the growing polyketide chain; an acyltransferase, which selectively binds an extender unit and trans-esterifies the extender onto an acyl carrier protein (ACP); the ACP serves as the acceptor for the extender unit and the growing polyketide chain. A type I PKS may further comprise additional domains, such as a ketoacyl reductase (KR), which reduces the β-ketone to a hydroxyl group, a dehydratase (DH) which removes the hydroxyl group and creates a double bond between the α and β carbon, an enoyl reductase (ER) which reduces the double bond generated by the DH domain, and a thioesterase (TE) terminating the synthesis process. A type I PKS may be an iterative PKS, catalyzing multiple elongation cycles repeatedly using the same functional domains, or it may be an assembly-line PKS comprising different modules, each of which catalyzes one elongation step (Hertweck 2009, Cogan *et al.* 2021). "Polyketide synthesis" as used herein includes synthesis of diketides and oligoketides.

The terms "polypeptide" and "protein" are used interchangeably herein. Naturally, protein functionality, including enzymatic activity, is dependent on further factors, such as temperature, pH, salts, and the like and may require further proteins and/or co-factors as it is known to the skilled person. Polypeptides disclosed herein may start with an additional methionine residue resulting from translation of the start codon, or alternatively with a different additional residue, such as valine or leucine resulting from translation of an alternative start codon, likewise polypeptides disclosed herein as beginning with a methionine residue may also be present without said methionine e.g. if used in context of an N-terminal fusion to a tag or other polypeptide.

### Brief description of the Drawings

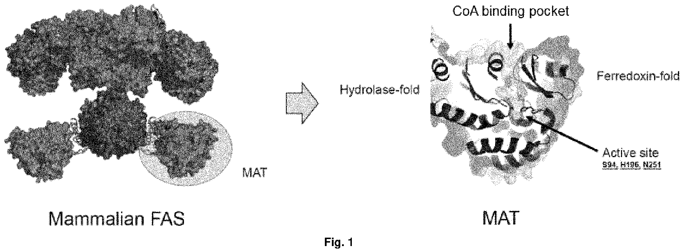

**Figure 1 (Fig. 1****)** shows a cartoon of the structure of a mammalian FAS homodimer with one of the malonyl-acetyl-transferases (MAT) highlighted as an exemplary AT, together with a corresponding cartoon showing the position of the CoA binding channel within a MAT as a cartoon. The numbering of the residues of the active site (S94, H196 and N251) is based on SEQ ID NO: 29.

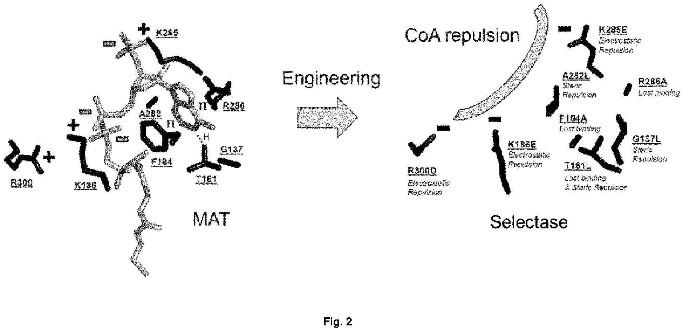

**Figure 2 (Fig. 2****)** shows the residues of a murine MAT (SEQ ID NO: 29) as an exemplary acyltransferase. Structural sketches of the eight different identified residues are shown in black with the positively charged residues (K186, K285 and R300) being marked with a black "+". A structural sketch of the CoA is shown in gray, with the negatively charged phosphate groups being marked with a gray "-". Pi-stacking between the CoA-Adenine and the residues F184 and R286 are indicated as "π". H-bonding between residue T161 and the CoA-Adenine is indicated with a dotted line and an "H".

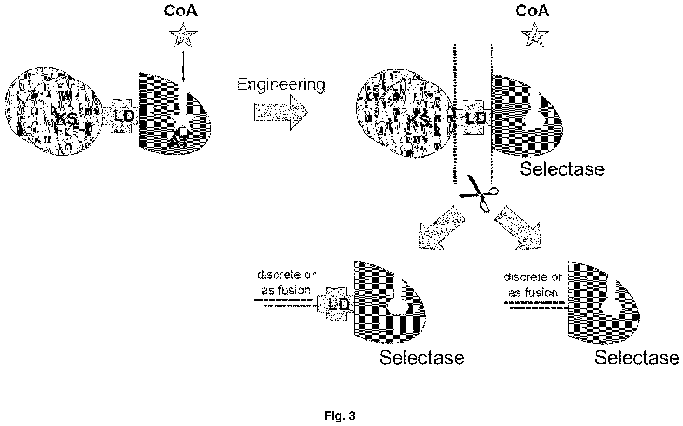

**Figure 3 (Fig. 3****)** shows an exemplary scheme of engineering an acyltransferase into an altered acyltransferase having coenzyme specificity. In the top left is a sketch of a KS-LD-AT polypeptide capable of binding CoA. In the top right is a sketch of an altered KS-LD-AT according to the present invention. On the bottom right are examples of using an altered acyltransferase of the present invention.

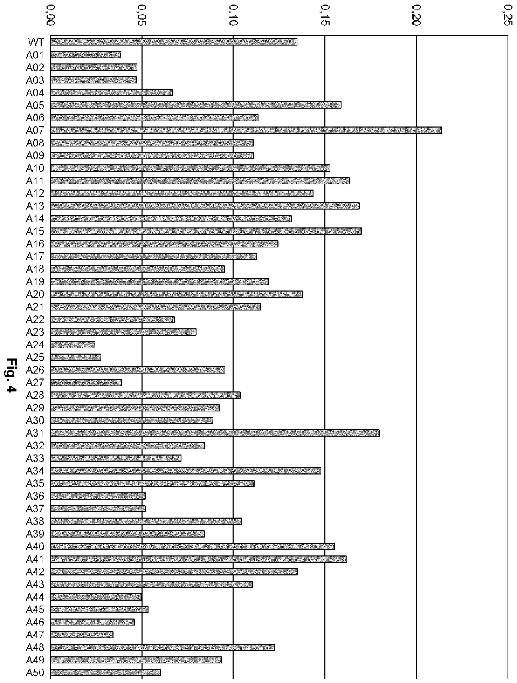

**Figure 4 (Fig. 4****)** shows turnover rates for the transfer of malonyl moieties from a non-CoA substrate. Specific transfers from the non-CoA substrate S-Malonyl-N-hexanoylcysteamine to N-(2-hydroxyethyl)-hexanamide were analyzed at fixed substrate and acceptor concentrations. The Y-axis shows enzyme-specific turnover in s⁻¹. Measurements were performed in duplets.

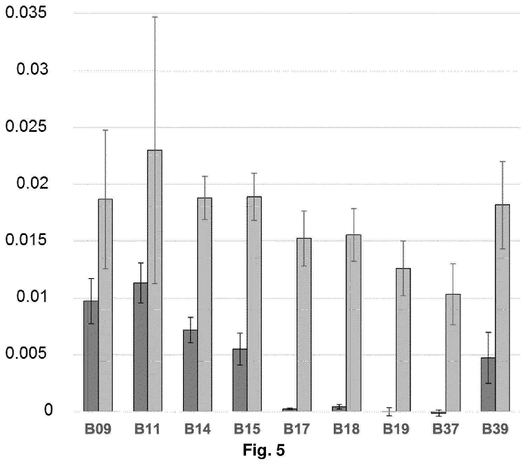

**Figure 5 (Fig. 5****)** shows turnover rates for the transfer of methylmalonyl moieties from methylmalonyl-CoA (black) or S-Methylmalonyl-N-hexanoylcysteamine (gray) to ACP6. The Y-axis shows enzyme-specific turnover in s⁻¹. Measurements were performed in at least triplets.

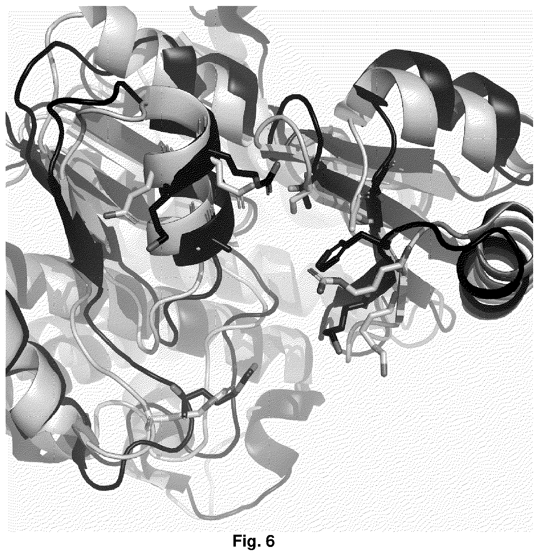

**Figure 6 (Fig. 6****)** shows a structural alignment of DEBS AT6 in grey (PDB code: 8g4u) and mouse MAT in black (PDB code: 5my0). The alignment was performed with Pymol (and was specified to the range of the 8 reference positions).

### Detailed description

The present invention will now be described in detail based on the detailed description, including non-limiting Examples, the attached drawings and the sequences as provided with the sequence listing.

In a first aspect, there is provided a method comprising: (a) providing (i) at least one non-CoA acyltransferase substrate; and (ii) at least one altered acyltransferase ; and (iii) at least one acyl carrier protein (ACP); (b) allowing the at least one acyltransferase to transfer the acyl group of the at least one non-CoA acyltransferase substrate to the at least one ACP; (c) obtaining at least one acyl-ACP; wherein the at least one altered acyltransferase has a CoA-binding pocket that is altered to reduce CoA acyltransferase substrate transfer activity.

In one embodiment of the first aspect, step (b) is performed in at least one cell comprising the components (i), (ii), and (iii) of step (a).

In one embodiment of the first aspect, step (b) is performed outside of a cell.

In one embodiment of the first aspect, step (b) the at least one altered acyltransferase is provided as a purified or partially purified protein or as a lysate comprising the altered acyltransferase, optionally wherein the at least one altered acyltransferase is provided as a fusion protein further comprising at least one linker domain and optionally at least one ketoacyl synthase domain.

In a second aspect, there is provided a method for polyketide synthesis, the method comprising: (a) providing (i) at least one non-CoA acyltransferase substrate; and (ii) optionally at least one CoA acyltransferase substrate; and (ii) a polyketide synthase (PKS) system, wherein the PKS system comprises at least one altered acyltransferase; (b) allowing polyketide synthesis, wherein the at least one non-CoA acyltransferase substrate, and optionally the at least one CoA acyltransferase substrate, is/are used as a substrate in the polyketide synthesis; (c) obtaining at least one polyketide, and optionally purifying said at least one polyketide; wherein the at least one altered acyltransferase has a CoA-binding pocket that is altered to reduce CoA acyltransferase substrate transfer activity.

In one embodiment of the first or second aspect, the at least one altered acyltransferase comprises one or more amino acid exchange(s) at reference position(s) (i) T161 according to SEQ ID NO: 29, or an analogous amino acid, such as a threonine, serine or asparagine residue, at said reference position as defined in Table 1, wherein the amino acid is exchanged to alanine, glycine, valine, leucine, isoleucine, tyrosine, phenylalanine, tryptophan, aspartate or glutamate; and/or (ii) F184 according to SEQ ID NO: 29, or an analogous amino acid, such as a phenylalanine residue, at said reference position as defined in Table 1, wherein the amino acid is exchanged to alanine, glycine, valine, leucine, isoleucine, methionine, aspartate or glutamate; and/or (iii) R286 according to SEQ ID NO: 29, or an analogous amino acid, such as an arginine residue, at said reference position as defined in Table 1, wherein the amino acid is exchanged to alanine, glycine, valine, leucine, isoleucine, methionine, aspartate or glutamate; and/or (iv) K186 according to SEQ ID NO: 29, or an analogous amino acid, such as a lysine or arginine residue, at said reference position as defined in Table 1, wherein the amino acid is exchanged to a negatively charged amino acid or to glycine, alanine, valine, leucine, isoleucine, methionine, phenylalanine, tyrosine, tryptophan, cysteine, serine, threonine, histidine or proline; and/or (v) K285 according to SEQ ID NO: 29, or an analogous amino acid, such as a lysine or arginine residue, at said reference position as defined in Table 1, wherein the amino acid is exchanged to a negatively charged amino acid; and/or (vi) R300 according to SEQ ID NO: 29, or an analogous amino acid, such as a lysine or arginine residue, at said reference position as defined in Table 1, wherein the amino acid is exchanged to a negatively charged amino acid, and/or (vii) A282 according to SEQ ID NO: 29, or an analogous amino acid, such as an alanine residue, at said reference position as defined in Table 1, wherein the amino acid is exchanged to valine, leucine, isoleucine, phenylalanine or tyrosine; and/or (viii) G137 according to SEQ ID NO: 29, or an analogous amino acid, such as a glycine residue, at said reference position as defined in Table 1, wherein the amino acid is exchanged to valine, leucine, isoleucine, phenylalanine or tyrosine.

Wild type (i.e. without comprising the one or more amino acid exchanges of the present invention) FAS and PKS acyltransferases can be polyspecific for multiple different acyl groups or specific for a particular acyl group. However, all of them are specific for CoA-bound substrates. The two different types of specificity, the CoA specificity and the acyl specificity, are conferred by different residues of the enzyme. The residues at the reference positions are crucial for binding to the CoA moiety of the substrate and the amino acid exchanges of the present invention can be used to alter these binding properties and alter acyltransferases so that they can bind to substrates covalently bound to moieties other than CoA, including synthetic coenzyme moieties. Acyltransferases having altered substrate specificity according to the present invention are acyltransferases having a reduced ability, preferably essentially no ability, to transfer CoA-bound substrates, while the acyl specificity may be unaltered. In certain embodiments, the altered acyltransferase is an acyl specific altered acyltransferase. In preferred embodiment, the at least one altered acyltransferase is an acyl polyspecific acyltransferase. As the at least one acyltransferase comprises specifically reprogrammed coenzyme-binding properties due to the altered CoA-binding region without the necessity for exchanging amino acid residues involved in the acyl moiety binding, the at least one altered acyltransferase of the present invention can retain the natural polyspecificity regarding the acyl moiety. Retaining the acyl polyspecificity can be of great importance for methods according to the first or second aspect, e.g. for using one acyltransferase for different acyl-moieties as substrates, in particular when using at least two different substrates for polyketide synthesis, fatty acid synthesis and/or non-ribosomal peptide synthesis.

The residues at reference positions (i), (ii) and (iii) are crucial residues for the binding pocket of the respective coenzyme (c.f. Fig. 2 for MAT as exemplary AT) and interact with the nucleobase portion of the CoA moiety through hydrogen bonding (reference position (i)) or pi-stacking (reference positions (ii) and (iii)). One or more amino acid exchange(s) at reference positions (i), (ii) and/or (iii) to alanine, glycine and/or valine may be used to prevent the H-bonding and/or Pi-stacking. An amino acid exchange at reference position (i) to leucine, isoleucine, tyrosine, phenylalanine, tryptophan, aspartate or glutamate may be used to sterically manipulate and reprogram the binding to coenzyme moieties, wherein an exchange to tyrosine, phenylalanine or tryptophan further may be used to stabilize the protein through pi-stacking. The amino acid exchange(s) at reference position (ii) and/or (iii) to leucine, isoleucine, methionine, aspartate or glutamate may be used to sterically manipulate the binding to coenzyme moieties.

Lysine or arginine residues at reference positions (iv), (v) and (vi) interact electrostatically with the negatively charged phosphate groups of CoA. Exchanging one or more residues at reference positions (iv), (v) and/or (vi) to one or more negatively charged residue(s) may be used to alter acyltransferases so that they, instead, bind to one or more positively charged chemical groups of coenzyme moieties other than CoA, including synthetic coenzyme moieties.

An amino acid exchange at reference position (iv) to amino acid residues that are, at a neutral pH, not positively charged or uncharged, and optionally non-polar, such as alanine, glycine, valine, leucine, isoleucine, methionine, phenylalanine, tyrosine, tryptophan, cysteine, serine, threonine, histidine, proline, in particular alanine, glycine, valine, leucine, isoleucine or methionine may be used to artificially eliminate the electrostatic interaction at this reference position. Experimental results show that the effect of amino acid exchanges at reference position (iv) are highly additive with amino acid exchanges at reference position, (i), (ii), (iii) and (vi) and suggest that the most important factor in respect to reference position (iv) is the elimination of the naturally occurring positive charge at this position, while the exact nature of the amino acid exchange or the introduction of a negative charge at this position are less relevant than the elimination of the positive charge.

An alanine residue at reference position (vii) and a glycine residue at reference position (viii) form structural parts of the coenzyme binding pocket. Exchanging the residues at one and/or both of these positions may be used to manipulate the structural shape of the coenzyme binding pocket through modification of the steric arrangement.

Preferred positions for altering an AT of the present invention are reference positions (i), (ii), (iii), (iv), (v) and/or (vi), alone or in combination, even more preferred is a combination of reference positions (ii) and (iii), preferably in combination with at least one further amino acid exchange at at least one other position, or a combination of reference positions (i) and (iv), preferably in combination with at least one further amino acid exchange at at least one other position, or a combination of reference positions (iv) and (vi), preferably in combination with at least one further amino acid exchange at least one other position.

In some embodiments of the first or second aspect, an amino acid exchange at reference position (i) is an exchange to leucine, alanine, tryptophan, or valine, preferably to leucine or alanine, more preferably to leucine; and/or wherein an amino acid exchange at reference position (ii) is an exchange to leucine, alanine, glycine, valine, or glutamate, preferably to leucine, alanine or glutamate, more preferably to leucine or alanine; and/or wherein an amino acid exchange at reference position (iii) is an exchange to leucine, alanine, glycine, or valine, preferably to leucine or alanine; and/or wherein an amino acid exchange at reference position (iv) is an exchange to a negatively charged amino acid, or to alanine or valine, preferably to glutamate or aspartate; and/or wherein an amino acid exchange at reference position (v) is an exchange to glutamate or aspartate; and/or wherein an amino acid exchange at reference position (vi) is an exchange to a negatively charged amino acid, or to alanine or valine, preferably to glutamate or aspartate; and/or wherein an amino acid exchange at reference position (vii) is an exchange to leucine, isoleucine, phenylalanine or tyrosine; and/or wherein an amino acid exchange at reference position (viii) is an exchange to leucine, isoleucine, phenylalanine or tyrosine, preferably to leucine.

In some embodiments of the first or second aspect, an amino acid exchange at reference position (i) is an exchange to leucine, isoleucine, tyrosine, phenylalanine or glycine; and/or wherein an amino acid exchange at reference position (ii) is an exchange to leucine, alanine, isoleucine or methionine; and/or wherein an amino acid exchange at reference position (iii) is an exchange to leucine, isoleucine or methionine; and/or wherein an amino acid exchange at reference position (iv) is an exchange to alanine, glycine, valine, leucine, isoleucine or methionine; and/or wherein an amino acid exchange at reference position (vi) is an exchange to alanine, glycine, valine, leucine, isoleucine or methionine and/or wherein an amino acid exchange at reference position (vii) is an exchange to valine; and/or wherein an amino acid exchange at reference position (viii) is an exchange to valine.

In some embodiments of the first or second aspect, the at least one altered acyltransferase comprises amino acid exchanges as defined above at reference positions (iv) and (i), or (iv) and (ii), or (iv) and (iii), or (iv) and (v), or (iv) and (vi), or (iv) and (vii), or (iv) and (viii), or (iv) and two or more of reference positions of (i), (ii), (iii), (v), (vi), (vii) and/or (viii), optionally wherein the amino acid exchange at reference position (iv) is an amino acid exchange to glutamate or aspartate.

In some embodiments of the first or second aspect, the at least one altered acyltransferase comprises amino acid exchanges as defined above at reference positions (i) and (ii), or (i) and (iii), or (i) and (iv), or (i) and (v), or (i) and (vi), or (i) and (vii), or (i) and (viii), or (i) and two or more of reference positions of (ii), (iii), (iv), (v), (vi), (vii) and/or (viii), optionally wherein the amino acid exchange at reference position (i) is an amino acid exchange to leucine, alanine, tryptophan, or valine, preferably to leucine or alanine, more preferably to leucine.

In preferred embodiments of the first or second aspect, the at least one altered acyltransferase comprises amino acid exchanges as defined above at reference positions (i), (ii), (iii), (iv), and optionally also at reference position (vi).

In some embodiments of the first or second aspect, the at least one altered acyltransferase comprises amino acid exchanges as defined above at reference positions: (i), optionally wherein the amino acid exchange at reference position (i) is an amino acid exchange to leucine, alanine, tryptophan, or valine, preferably to leucine or alanine, more preferably to leucine.

In some embodiments of the first or second aspect, the at least one altered acyltransferase further comprises amino acid exchanges as defined above at reference positions: (ii), optionally wherein the amino acid exchange at reference position (ii) is an amino acid exchange to leucine, alanine, glycine, valine, or glutamate, preferably to leucine, alanine or glutamate, more preferably to leucine or alanine; and (iii), optionally wherein the amino acid exchange at reference position (iii) is an amino acid exchange to leucine, alanine, glycine, or valine, preferably to leucine or alanine.

In some embodiments of the first or second aspect, the at least one altered acyltransferase further comprises an amino acid exchange as defined above at reference (iv), optionally wherein the amino acid exchange at reference position (iv) is an amino acid exchange to glutamate or aspartate.

In some embodiments of the first or second aspect, the at least one altered acyltransferase further comprises an amino acid exchange as defined above at reference (vi), optionally wherein the amino acid exchange at reference position (vi) is an amino acid exchange to glutamate or aspartate.

In some embodiments of the first or second aspect, the at least one altered acyltransferase comprises amino acid exchanges to glutamate at reference positions (iv) and (vi), or amino acid exchanges to aspartate at reference positions (iv) and (vi), or an amino acid exchange to glutamate at reference position (iv) and an amino acid exchange to aspartate at reference position (vi), or an amino acid exchange to aspartate at reference position (iv) and an amino acid exchange to glutamate at reference position (vi).

In one embodiment of the first or second aspect, the at least one altered acyltransferase comprises an amino acid exchange to aspartate at reference position (i) and an amino acid exchange to glutamate at reference position (ii).

In one embodiment of the first aspect, the at least one altered acyltransferase comprises an amino acid exchange to leucine at least at reference positions (i), (ii) and (iii).

In one embodiment of the first or second aspect, the at least one altered acyltransferase comprises an amino acid exchange to alanine at reference positions (i), (ii) and (iii), and an amino acid exchange to aspartate or glutamate at reference position (iv).

In one embodiment of the first or second aspect, the at least one altered acyltransferase comprises an amino acid exchange to alanine at reference positions (i), (ii) and (iii); and an amino acid exchange to aspartate at reference position (iv) and an amino acid exchange to aspartate at reference position (vi) or an amino acid exchange to aspartate at reference position (iv) and an amino acid exchange to glutamate at reference position (vi) or an amino acid exchange to glutamate at reference position (iv) and an amino acid exchange to aspartate at reference position (vi) or an amino acid exchange to glutamate at reference position (iv) and an amino acid exchange to glutamate at reference position (vi).

In one embodiment of the first or second aspect, the at least one altered acyltransferase comprises an amino acid exchange to leucine at reference positions (i), (ii) and (iii); and an amino acid exchange to glutamate or aspartate at reference position (iv).

In one embodiment of the first or second aspect, the at least one altered acyltransferase comprises an amino acid exchange to leucine at reference positions (i), (ii) and (iii); and an amino acid exchange to aspartate at reference position (iv) and an amino acid exchange to aspartate at reference position (vi) or an amino acid exchange to aspartate at reference position (iv) and an amino acid exchange to glutamate at reference position (vi) or an amino acid exchange to glutamate at reference position (iv) and an amino acid exchange to aspartate at reference position (vi) or an amino acid exchange to glutamate at reference position (iv) and an amino acid exchange to glutamate at reference position (vi).

In one embodiment of the first or second aspect, the at least one altered acyltransferase comprises an amino acid exchange to leucine at reference positions (i) and an amino acid exchange to alanine at reference positions (ii) and (iii); and an amino acid exchange to glutamate or aspartate at reference position (iv).

In one embodiment of the first or second aspect, the at least one altered acyltransferase comprises an amino acid exchange to leucine at reference positions (i) and an amino acid exchange to alanine at reference positions (ii) and (iii); and an amino acid exchange to aspartate at reference position (iv) and an amino acid exchange to aspartate at reference position (vi) or an amino acid exchange to aspartate at reference position (iv) and an amino acid exchange to glutamate at reference position (vi) or an amino acid exchange to glutamate at reference position (iv) and an amino acid exchange to aspartate at reference position (vi) or an amino acid exchange to glutamate at reference position (iv) and an amino acid exchange to glutamate at reference position (vi).

In one embodiment of the first or second aspect, the at least one altered acyltransferase comprises an amino acid exchange to leucine at reference positions (i) and an amino acid exchange to glutamate at reference position (ii) and an amino acid exchange to alanine at reference position (iii); and an amino acid exchange to glutamate or aspartate at reference position (iv).

In one embodiment of the first or second aspect, the at least one altered acyltransferase comprises an amino acid exchange to leucine at reference positions (i) and an amino acid exchange to glutamate at reference position (ii) and an amino acid exchange to alanine at reference position (iii); and an amino acid exchange to aspartate at reference position (iv) and an amino acid exchange to aspartate at reference position (vi) or an amino acid exchange to aspartate at reference position (iv) and an amino acid exchange to glutamate at reference position (vi) or an amino acid exchange to glutamate at reference position (iv) and an amino acid exchange to aspartate at reference position (vi) or an amino acid exchange to glutamate at reference position (iv) and an amino acid exchange to glutamate at reference position (vi).

In one embodiment of the first or second aspect, the at least one altered acyltransferase comprises an amino acid exchange to alanine at reference positions (i) and an amino acid exchange to glutamate at reference position (ii) and an amino acid exchange to alanine at reference position (iii); and an amino acid exchange to glutamate or aspartate at reference position (iv).

In one embodiment of the first or second aspect, the at least one altered acyltransferase comprises an amino acid exchange to alanine at reference positions (i) and an amino acid exchange to glutamate at reference position (ii) and an amino acid exchange to alanine at reference position (iii); and an amino acid exchange to aspartate at reference position (iv) and an amino acid exchange to aspartate at reference position (vi) or an amino acid exchange to aspartate at reference position (iv) and an amino acid exchange to glutamate at reference position (vi) or an amino acid exchange to glutamate at reference position (iv) and an amino acid exchange to aspartate at reference position (vi) or an amino acid exchange to glutamate at reference position (iv) and an amino acid exchange to glutamate at reference position (vi).

In one embodiment of the first or second aspect, the at least one altered acyltransferase comprises an amino acid exchange to aspartate at reference positions (i) and an amino acid exchange to glutamate at reference position (ii) and an amino acid exchange to alanine at reference position (iii); and an amino acid exchange to glutamate or aspartate at reference position (iv).

In one embodiment of the first or second aspect, the at least one altered acyltransferase comprises an amino acid exchange to aspartate at reference positions (i) and an amino acid exchange to glutamate at reference position (ii) and an amino acid exchange to alanine at reference position (iii); and an amino acid exchange to aspartate at reference position (iv) and an amino acid exchange to aspartate at reference position (vi) or an amino acid exchange to aspartate at reference position (iv) and an amino acid exchange to glutamate at reference position (vi) or an amino acid exchange to glutamate at reference position (iv) and an amino acid exchange to aspartate at reference position (vi) or an amino acid exchange to glutamate at reference position (iv) and an amino acid exchange to glutamate at reference position (vi).

In one embodiment of the first or second aspect, the at least one altered acyltransferase comprises an amino acid exchange to aspartate at reference positions (i) and an amino acid exchange to alanine at reference position (ii) and an amino acid exchange to alanine at reference position (iii); and an amino acid exchange to glutamate or aspartate at reference position (iv).

In one embodiment of the first or second aspect, the at least one altered acyltransferase comprises an amino acid exchange to aspartate at reference positions (i) and an amino acid exchange to alanine at reference position (ii) and an amino acid exchange to alanine at reference position (iii); and an amino acid exchange to aspartate at reference position (iv) and an amino acid exchange to aspartate at reference position (vi) or an amino acid exchange to aspartate at reference position (iv) and an amino acid exchange to glutamate at reference position (vi) or an amino acid exchange to glutamate at reference position (iv) and an amino acid exchange to aspartate at reference position (vi) or an amino acid exchange to glutamate at reference position (iv) and an amino acid exchange to glutamate at reference position (vi).

In one embodiment of the first or second aspect, the at least one altered acyltransferase comprises an amino acid exchange to alanine at reference positions (i) and an amino acid exchange to glutamate at reference position (ii) and an amino acid exchange to aspartate at reference position (iii); and an amino acid exchange to glutamate or aspartate at reference position (iv).

In one embodiment of the first or second aspect, the at least one altered acyltransferase comprises an amino acid exchange to alanine at reference positions (i) and an amino acid exchange to glutamate at reference position (ii) and an amino acid exchange to aspartate at reference position (iii); and an amino acid exchange to aspartate at reference position (iv) and an amino acid exchange to aspartate at reference position (vi) or an amino acid exchange to aspartate at reference position (iv) and an amino acid exchange to glutamate at reference position (vi) or an amino acid exchange to glutamate at reference position (iv) and an amino acid exchange to aspartate at reference position (vi) or an amino acid exchange to glutamate at reference position (iv) and an amino acid exchange to glutamate at reference position (vi).

In preferred embodiments of the first or second aspect, the at least one altered acyltransferase is at least one isolated acyltransferase originating from a FAS or PKS.

In one embodiment of the first or second aspect, the at least one isolated acyltransferase is a polypeptide or part of a polypeptide comprising or consisting of the acyltransferase, without comprising further parts and/or domains, including a LD, of the FAS or PKS from which it originates. The isolated acyltransferase may be part of a fusion protein according.

In another embodiment of the first or second aspect, the at least one isolated acyltransferase is part of a polypeptide comprising or consisting of the acyltransferase and a LD, or a part thereof, without comprising further parts and/or domains of the FAS or PKS from which it originates. The isolated acyltransferase may be part of a fusion protein according to the second aspect.

In another embodiment of the first or second aspect, the at least one isolated acyltransferase is part of a polypeptide comprising or consisting of the portion of a FAS or PKS spanning the ketoacyl synthase (KS), the acyltransferase and the LD connecting the KS and the acyltransferase, without comprising further parts and/or domains of the FAS or PKS from which it originates. Such a KS-LD-acyltransferase may be part of a fusion.

In certain embodiments of the first or second aspect, the at least one altered acyltransferase is provided as at least one fusion protein comprising at least one altered acyltransferase according to the present invention, preferably wherein the fusion protein further comprises at least one linker domain and optionally at least one ketoacyl synthase domain.

In some embodiments of the first or second aspect, the at least one fusion protein may comprise a tag for purification, for example but not limited to at least one His-tag, step-tag, FLAG-tag, myc-tag, MBP-tag, GST-tag, or a combination thereof.

The acyltransferase of the present invention of preferably linked to other functional PKS or FAS parts and/or domains, in particular a ketoacyl synthase domain, via a FAS and/or PKS LD. Of course, the acyltransferase may also be linked to other functional parts and/or domains by conventional polypeptide linkers known in the art, such as glycine linkers, GS linkers, PT-linkers or other commonly used linkers.

In some embodiments of the first or second aspect, the at least one fusion protein may be a polyketide synthase (PKS), or a part thereof, in which at least one acyltransferase has been replaced by an acyltransferase of the present invention, wherein the PKS portion of the fusion protein may comprise further engineered modifications.

In one embodiment of the first aspect, the acyltransferase originates from a FAS. In certain embodiments, the AT originates form a FAS of an organism selected from vertebrata, including aves and mammalia, including artiodactyla, perissodactyla, carnivora, primates and rodentia, preferably wherein the acyltransferase originates from a fatty acid synthase comprising or consisting of a wild type amino acid sequence selected from any one of SEQ ID NO: 1 to 28, or a sequence having at least 80%, 81%, 82%, 83%, 84%, 85%, 86%, 87%, 88%, 89%, 90%, 91%, 92%, 93%, 94%, 95%, 96%, 97%, 98%, or at least 99% sequence identity thereto.

In some embodiments of the first aspect, the altered acyltransferase comprises or consists of an amino acid sequence of any of SEQ ID NOs: 57 to 82 or 111 to 133 or 154 to 165, or a sequence having at least 80%, 81%, 82%, 83%, 84%, 85%, 86%, 87%, 88%, 89%, 90%, 91%, 92%, 93%, 94%, 95%, 96%, 97%, 98%, or at least 99% sequence identity thereto. SEQ ID NOs: 57 to 82 and 111 to 154 are exemplary mutants of the mouse MAT. SEQ ID NOs: 154 to 165 are exemplary mutants of *Saccharopolyspora erythraea* DEBSAT6.

In one embodiment of the second aspect, the PKS system is a PKS type I system, wherein one, two, three or more PKS modules comprise and/or interact with at least one altered acyltransferase.

In one embodiment of the second aspect, the PKS system is a PKS type II system, wherein one, two, three or more PKS modules interact with at least one altered acyltransferase.

Usually, the starter unit or the extender unit is a CoA-bound acyl group, such as acetyl-CoA, malonyl-CoA, methylmalonyl-CoA or propionyl-CoA, which are selectively recognized and transferred to the ACP by the acyltransferase. By exchanging the acyltransferase to an altered acyltransferase of the present invention, a PKS system may be reprogrammed to incorporate starter and/or extender units bound to other coenzyme moieties, including synthetic coenzyme moieties. The acyltransferase of a PKS may be replaced by an altered acyltransferase of the present invention through regular cloning strategies available to the skilled person.

In modular, assembly-line PKS systems, the acyltransferase of one, or two, or more, or all modules may be replaced by an altered acyltransferase of the present invention, optionally including the adjacent LD, or a part thereof, and/or KS, to produce a reprogrammed PKS system capable of using starter and/or extender units bound by coenzyme moieties other than CoA.

In one embodiment of the first or second aspect, the at least one altered acyltransferase is part of at least one multifunctional PKS type I polypeptide, optionally through replacement of at least one of the PKS acyltransferases.

In one embodiment of the second aspect, the at least one altered acyltransferase is provided as at least one seperate protein, optionally as a fusion protein as defined above, comprising the ketoacyl synthase domain of the FAS or PKS from which the altered acyltransferase originates and/or the linker domain of the FAS or PKS from which the altered acyltransferase originates or a different linker as described herein or known in the art.

In one embodiment of the second aspect, the first, second, third, fourth, fifth, six, seventh, eighth, ninth, tenth, eleventh, thirteenth, fourteenths, fifteenth, and/or sixteenth PKS module and/or one or more subsequent PKS modules comprise(s) and/or interact(s) with at least one altered acyltransferase. In a PKS module comprising at least one altered acyltransferase, the original acyltransferase of that PKS module may be replaced by an altered acyltransferase.

In preferred embodiments, the cell comprises a PKS system in which at least one acyltransferase has been replaced with an altered acyltransferase of the present invention. In certain embodiments, the cell comprises a modular type I PKS system in which the acyltransferase of one, ortwo, or more, or all PKS modules has been replaced by an altered acyltransferase optionally including the adjacent LD, or a part thereof, and/or KS, of the FAS or PKS from which the altered acyltransferase originates.

In embodiment, replacement of a PKS acyltransferase with an altered acyltransferase may be the replacement of only the original acyltransferase with only the altered acyltransferase.

In another embodiment, replacement of a PKS acyltransferase with an altered acyltransferase may be the replacement of the original acyltransferase and the adjacent linker domain of that PKS with the altered acyltransferase and adjacent linker domain of the FAS or PKS from which the altered acyltransferase originates or a different linker as described herein or known in the art.

In another embodiment, replacement of a PKS acyltransferase with an altered acyltransferase may be the replacement of the original acyltransferase, the adjacent linker domain and adjacent ketoacyl synthase domain of that PKS with the altered acyltransferase, the ketoacyl synthase domain of the FAS or PKS from which the altered acyltransferase originates and the linker domain of the FAS or PKS from which the altered acyltransferase originates or a different linker as described herein or known in the art.

In one embodiment of the second aspect, the at least one altered acyltransferase is provided as a separate polypeptide, optionally as a fusion protein further comprising at least one linker domain and optionally at least one ketoacyl synthase domain.

In one embodiment of the second aspect, the polyketide synthesis, or part of the polyketide synthesis, is performed in at least one cell comprising the PKS system or parts thereof.

In embodiments of the first or second aspect, relating to performing the method or parts thereof inside a at least one cell, the at least one altered acyltransferase may be provided as at least one a nucleic acid encoding the at least one altered acyltransferase or a fusion protein comprising the same.

The nucleic acid may be double and/or single stranded DNA and/or RNA, including mRNA. DNA and RNA, including naturally occurring nucleosides / nucleotides, modified and/or non-natural nucleosides / nucleotides and natural and artificial (phosphothioate) linkages of nucleosides / nucleotides. A nucleic acid that is not part of an expression construct or vector may comprise other sequences and/or modification for improving the stability. In certain embodiments, the nucleic acid is comprised by an expression construct or vector comprising at least one nucleic acid of the third aspect.

The nucleic acid, expression construct or vector may further comprise sequences for expression in the desired host cell, such as a promoter, including inducible promoters, and/or a transcription terminator operably linked to the nucleic acid encoding the acyltransferase or the fusion protein, a selectable marker, a sequence for replication in a desired host cell, and/or further regulatory sequences, including sequences regulatory for transcription, RNA processing and/or stability, and/or translation. The nucleic acid or expression construct or vector may comprise a cDNA sequence, devoid of introns, encoding the acyltransferase and/or fusion protein of the present invention, e.g. for expression in prokaryotic cells. Recombinant protein expression, as well as suitable vectors and expression systems, promoters and further regulatory sequences are well-established for a multitude of cell types. The skilled person is well-aware of the particulars and requirements for recombinant protein expression in different cell types and can easily determine suitable components to choose for expression in the desired host cell. Vector backbones for an expression construct or vector may for example be pET22b, pET300, pET301, pET28b, pCDF, pKC1139, pETDuet or pGEX-6P-1 vector systems, but are not limited thereto.

The nucleic acid, expression construct or vector may be codon optimized, wherein the codon optimization may be adapted according to the desired host cell. Codon optimizations for different cell types are well-established in the art and tools for codon optimization are readily available to the skilled person.

The nucleic acid, expression construct or vector may be suitable for stable replication and maintenance in the desired host cell. The nucleic acid and/or expression construct or vector may further be suitable for integration into the host genome.

In one embodiment of the first or second aspect, at least one nucleic acid encoding the at least one altered acyltransferase or at least one fusion protein comprising the same may be stably integrated into the genome, including the chromosomal/nuclear genome and/or the plastid genome of the at least one cell, e.g. by any conventional genome editing strategy and/or any conventional vector suitable for genome integration. In certain embodiments, the at least one nucleic acid may be integrated into the genome by replacing an endogenous nucleic acid encoding an acyltransferase.

In another embodiment of the first or second aspect, at least one endogenous gene of the at least one cell encoding an acyltransferase is mutated to encode an altered acyltransferase of the present invention, wherein the mutation may be performed by any mutation strategy, preferably a SDN-1 (site directed nuclease 1) mutation strategy.

In another embodiment of the first or second aspect, at least one nucleic acid and/or at least one expression construct or vector encoding the at least one altered acyltransferase or at least one fusion protein encoding the same is present extrachromosomally in the at least one cell.

In one embodiment, at least one endogenous acyltransferase of the at least one cell is knocked out, knocked down or otherwise inactivated and complemented with the at least one altered acyltransferase and/or fusion protein comprising the same.

In one embodiment of the second aspect, the polyketide synthesis, or part of the polyketide synthesis, is performed outside of a cell.

An altered acyltransferase or fusion protein comprising the same of the various embodiments of the first aspect, may be purified using protein purification methods disclosed herein or any other conventional protein purification methods.

The at least one altered acyltransferase of the present invention may be expressed in any cellular expression system, established systems include prokaryotic cells, including Escherichia coli strains, such as but not restricted to various different well-established BL21 or BL21 (DE3) expression strains as well as derivatives thereof, including for example Rosetta, BAP1 or NiCo21 (DE3) strains, other E. coli strains, such as E. coli K12 strain JM83 or other prokaryotic cells such as Bacillus subtilis expression systems, eukaryotic expression systems, for example including Saccharomyces cerevisiae cells, Komagataella phaffii (formerly called Pichia pastoris) cells, Aspergillus niger cells, or immortalized cell lines, such as insect cell expression systems, for example Sf-9 or Sf-21 cells, or mammalian cell lines, such Chinese hamster Ovary (CHO) cells, HEK 293 cells and the like.

In one embodiment of the second aspect, the at least one altered acyltransferase is provided as a purified or partially purified protein or as a lysate comprising the altered acyltransferase, optionally wherein the at least one altered acyltransferase is provided as a fusion protein further comprising at least one linker domain and optionally at least one ketoacyl synthase domain.

In one embodiment of the first or second aspect, the CoA acyltransferase substrate transfer activity is reduced to ≤50%, ≤40%, or ≤30%, preferably to ≤20% or ≤10%, more preferably to ≤5%, ≤2%, most preferably ≤1%, In one embodiment, the substrate transfer activity is measured and compared as Vmax (maximum velocity). In another embodiment, the substrate transfer activity is measured and compared as K_{cat} / K_{M} (specificity constant).

In one embodiment of the first or second aspect, the CoA acyltransferase substrate transfer activity is ≤50%, ≤40%, or ≤30%, preferably ≤20% or ≤10%, more preferably ≤5%, ≤2%, or ≤1% of the transfer activity regarding the at least one non-CoA acyltransferase substrate provided in step (a). In one embodiment, the substrate transfer activity is measured and compared as Vmax (maximum velocity). In another embodiment, the substrate transfer activity is measured and compared as K_{cat} / K_{M} (specificity constant).

In one embodiment of the first or second aspect, wherein the at least one altered acyltransferase has a CoA-binding pocket that is altered to reduce CoA acyltransferase substrate transfer activity and to increase transfer activity for the at least one non-CoA acyltransferase substrate.

In one embodiment of the first or second aspect, the at least one altered acyltransferase has a CoA-binding pocket that is altered to transfer the at least one non-CoA acyltransferase substrate at a higher rate than the equivalent CoA acyltransferase substrate with the same acyl group and/or, in case at least one CoA acyltransferase substrate is provided in step (a), the at least one CoA acyltransferase substrate provided in step (a).

In one embodiment of the first or second aspect, the at least one non-CoA substrate comprises or consists of (ii) a non-CoA coenzyme; and (i) an acyl group; optionally wherein part (i) and part (ii) are linked via an ester, preferably a thioester. It is important that the non-CoA coenzyme and the carboxy group of the acyl group form a high energy bond, the cleavage of which drives the transfer reaction of the acyl group to the ACP to form acyl - ACP. Preferably the high energy bond is a thioester. Thus, the non-CoA coenzyme preferably has a thiol group to form a thioester with with the acyl group.

In one embodiment of the first or second aspect, the non-CoA coenzyme has a neutral net charge or a positive net charge. In one embodiment, the positive net charge is a single, two-fold, three-fold or four-fold positive net charge.

In one embodiment of the first or second aspect, the non-CoA coenzyme does not comprise a negative charge.

In one embodiment, the non-CoA coenzyme has a molar mass of ≤800 g/mol.

In one embodiment, the non-CoA coenzyme is a peptide. In certain embodiment of the method or parts thereof being performed inside at least one cell, the peptide may be encoded by at least one nucleic acid molecule that is translated inside the at least one cell. In certain embodiments, the peptide binds to the acyl group via an amino acid side chain, preferably via an thiol group of an amino acid side chain, for example the thiol group of cystein.

In one embodiment, the non-CoA acyltransferase substrate is provided as a complete preformed substrate. In another embodiment, the non-CoA acyltransferase substrate is provided as non-CoA coenzyme and carboxylic acid as separate molecules, which are reacted to form the at least one non-CoA acyltransferase substrate. In certain embodiment of the method or parts thereof being performed inside at least one cell, the non-CoA coenzyme and carboxylic acid may be introduced as separate molecules into the at least one cell and reacted to form the at least one non-CoA acyltransferase substrate within said at least one cell.

In one embodiment of the first or second aspect, the acyl group comprises or consist of a malonyl group, an acetyl group, a methylmalonyl group, a propionyl group, or a derivative thereof.

Starter substrates may fulfil the following formula:

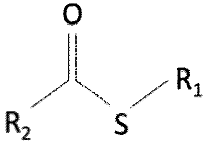

R1: non-CoA coenzyme, preferably ≤800 g/mol; R2: C1-C8 alkyl, C2-C8 alkenyl, C2-C8 alkynyl, cycloalkyl, heterocycloalkyl, benzyl, benzyl, heterobenzyl. All R2 may be functionalized at one or more positions with R3.

R3: halogen, alkyl, alkenyl, alkynyl, cycloalkyl, heterocycloalkyl, halogen, aryl, heteroaryl, benzyl, heterobenzyl, benzoyl, heterobenzoyl, -CN, -OCH3, -OR4, -C(=O)R4, -SR4, - S(=O)R4, -S(=O)2R4, -NO2, -NR4R5, -C(=O)OR4, -azide, -propargyl, -O(C=O)R4, - OC(=O)OR4, C(=O)NR4R5, -OC(=O)NR4R5. All R3 may be functionalized at one or more positions with R4.

R4: hydrogen, alkyl, alkenyl, alkynyl, cycloalkyl, heterocycloalkyl, aryl, heteroaryl, benzyl, heterobenzyl, benzoyl, heterobenzoyl. All R4 may be functionalized at one or more positions with R5.

R5: hydrogen, alkyl, alkenyl, alkynyl, cycloalkyl, heterocycloalkyl, aryl, heteroaryl, benzyl, heterobenzyl, benzoyl, heterobenzoyl. All R5 may be functionalized at one or more positions with R6.

R6: hydrogen, halogen, alkyl, alkenyl, alkynyl, aryl, heteroaryl, benzyl, heterobenzyl, benzoyl, heterobenzoyl, halogen, propargyl, azide, -CN, -NO2, -OH, -SH, -NH2, -COOH, - C(=O)H, -S(=O)H, -SO2H, -OCH3, SCH3.

Extender substrates may fulfil the following formula:

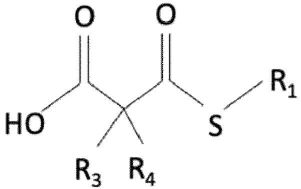

R1: non-CoA coenzyme, preferably ≤800 g/mol; R3, R4 = Cycloalkyl, heterocycloalkyl, All R3R4 may be functionalized at one or more positions with R4.

R3: hydrogen, halogen, alkyl, alkenyl, alkynyl, aryl, heteroaryl, benzyl, heterobenzyl, benzoyl, heterobenzoyl, halogen, -CN, -OCH3, -OR6, -C(=O)R6, -SR6, -S(=O)R6, - S(=O)2R6, -NO2, -NR6R7, -C(=O)OR6, -azide, -propargyl, -O(C=O)R6, -OC(=O)OR7, C(=O)NR6R7, -OC(=O)NR6R7, All R3 may be functionalized at one or more positions with R4.

R4 = as R3

R5: halogen, alkyl, alkenyl, alkynyl, aryl, heteroaryl, benzyl, heterobenzyl, benzoyl, heterobenzoyl, -azide, halogen, -CN, -OCH3, -OR6, -C(=O)R6, -SR6, -S(=O)R6, - S(=O)2R6, -NO2, -NR6R7, -C(=O)OR6, -azid, -propargyl, -O(C=O)R6, -OC(=O)OR7, C(=O)NR6R7, -OC(=O)NR6R7, All R5 may be functionalized at one or more positions with R8.

R6: hydrogen, alkyl, alkenyl, alkynyl, cycloalkyl, heterocycloalkyl, aryl, heteroaryl, benzyl, heterobenzyl, benzoyl, heterobenzoyl. All R3 may be functionalized at one or more positions with R8.

R7: hydrogen, alkyl, alkenyl, alkynyl, cycloalkyl, heterocycloalkyl, aryl, heteroaryl, benzyl, heterobenzyl, benzoyl, heterobenzoyl. All R7 may be functionalized at one or more positions with R8.

R8: hydrogen, halogen, alkyl, alkenyl, aryl, heteroaryl, benzyl, heterobenzyl, halogen, propargyl, -azid, -CN, -NO2, -OH, -SH, -NH2, -COOH, -C(=O)H, -S(=O)H, -SO2H, -OCH3, SCH3.

In one embodiment, the non-CoA acyltransferase substrate is 3-Oxo-3-[[2-[(1-oxohexyl)amino]ethyl]thio]-propanoic acid (N-hexanoylcysteamine).

In a third aspect, there is provided a use of at least altered acyltransferase as defined in the first or second aspect, wherein the at least one altered acyltransferase originates from a PKS; and/or at least one non-CoA acyltransferase substrate as defined in the second aspect, and optionally at least one CoA acyltransferase substrate for polyketide synthesis, fatty acid synthesis, and/or nonribosomal peptide synthesis.

In certain embodiments of the third aspect, the polyketide synthesis, fatty acid synthesis and/or non-ribosomal peptide synthesis may be a synthesis of polyketide, and/or fatty acid and/or protein hybrids with metabolites of other biosynthetic synthesis pathways.

In one embodiment, synthesis of the third aspect is in vivo synthesis in at least one cell comprising the at least one altered acyltransferase or fusion protein comprising the same as defined in the first or second aspect, further using at least one suitable medium and at least one non-CoA acyltransferase substrate as defined in the first or second aspect, the at least one cell thus representing an engineered production strain.

In another embodiment, synthesis of the third aspect is in vitro synthesis using at least one purified or partially purified altered acyltransferase or fusion protein comprising the same as defined in the first or second aspect, at least one non-CoA acyltransferase substrate as defined in the first or second aspect, optionally at least one CoA acyltransferase substrate and further optionally at least one buffer and/or further suitable reaction components.

In another embodiment, synthesis of the third aspect is in vitro synthesis using lysate of at least one cell comprising at least one altered acyltransferase or fusion protein comprising the same as defined in the first or second aspect, at least one non-CoA acyltransferase substrate as defined in the first or second aspect, optionally at least one CoA acyltransferase substrate and further optionally at least one buffer and/or further suitable reaction components.

In another embodiment, synthesis of the third aspect is in vitro synthesis using at least one in vitro transcription/translation system expressing at least one nucleic acid and/or at least one expression construct or vector encoding the at least one altered acyltransferase or fusion protein comprising the same as defined in the first or second aspect, at least one non-CoA acyltransferase substrate as defined in the first or second aspect, optionally at least one CoA acyltransferase substrate and further optionally at least one buffer and/or further suitable reaction components.

In another embodiment, synthesis of the third aspect is in vitro synthesis further using at least one purified or partially purified altered acyltransferase or fusion protein comprising the same as defined in the first or second aspect, at least one non-CoA acyltransferase substrate as defined in the first or second aspect, optionally at least one CoA acyltransferase substrate and further, optionally, at least one buffer and/or further suitable reaction components.

In another embodiment, synthesis of the third aspect is in vitro synthesis using lysate of at least one cell comprising the at least one altered acyltransferase or fusion protein comprising the same as defined in the first or second aspect, further using at least one non-CoA acyltransferase substrate as defined in the first or second aspect, optionally at least one CoA acyltransferase substrate, at least one buffer and optionally further suitable reaction components.

In another embodiment, synthesis of the third aspect is in vitro synthesis using at least one in vitro transcription/translation system expressing at least one nucleic acid and/or at least one expression construct or vector encoding the at least one altered acyltransferase or fusion protein comprising the same as defined in the first or second aspect, at least one non-CoA acyltransferase substrate as defined in the first or second aspect, optionally at least one CoA acyltransferase substrate and further optionally at least one buffer and/or further suitable reaction components.

In a fourth aspect, there is provided a kit comprising at least one altered acyltransferase as defined in the first or second aspect; and/or at least one non-CoA acyltransferase substrate as defined in the second aspect; optionally further comprising at least one CoA acyltransferase substrate, and/or at least one container, and/or a set of reagents including buffer and/or medium, and/or means of transfection. A kit will usually comprise all agents, buffers, co-factors etc. necessary to guarantee the functionality of the at least one acyltransferase in an assay of interest.

In one embodiment, the kit of the fourth aspect is a kit for in vivo synthesis in at least one cell.

In another embodiment, the kit of the fourth aspect comprises at least one purified or partially purified altered acyltransferase of the first or second aspect and/or at least one purified or partially purified fusion protein comprising the same, at least one non-CoA acyltransferase substrate, optionally at least one CoA acyltransferase substrate and further optionally at least one buffer and/or further suitable reaction components.

In another embodiment, the kit of the fourth aspect comprises lysate of at least one cell, comprising the at least one altered acyltransferase or fusion protein comprising the same as defined in the first or second aspect, the kit further comprising at least one non-CoA acyltransferase substrate as defined in the first or second aspect, optionally at least one CoA acyltransferase substrate, at least one buffer and optionally further suitable reaction components.

In another embodiment, the kit of the fourth aspect comprises at least one in vitro transcription/translation system expressing at least one nucleic acid and/or at least one expression construct or vector encoding the at least one altered acyltransferase or fusion protein comprising the same as defined in the first or second aspect, at least one non-CoA acyltransferase substrate as defined in the first or second aspect, optionally at least one CoA acyltransferase substrate and further optionally at least one buffer and/or further suitable reaction components.

### Examples

### Example 1: In silico design for targeted acyltransferase reprogramming

Due to their modular nature and their innate variability in acyl backbone reduction, PKS systems are an enticing enzymatic platform to be exploited for synthesizing a plethora of different useful biomolecules, in particular for drug design. A crucial aspect limiting a versatile and effective biotechnological use of PKS is the nature of naturally occurring acyltransferases, which have a relatively high specificity when it comes to the extender subunits (Smith and Tsai 2007). The MAT domains of metazoan FAS enzymes were shown to allow an efficient transfer of different acyl substrates (Rittner *et al.* 2018).

However, while a FAS MAT might therefore offer a certain versatility regarding the acyl group of the substrate, both FAS MATs and PKS ATs suffer from a crucial shortcoming: they are fully dependent on CoA-bound substrates. The dependency on Acyl-CoA substrates strongly limited the possibility for artificial biomolecule production using an engineered PKS system for *in vivo* synthesis. CoA-bound molecules, in particular Acetyl-CoA and Malonyl-CoA, are highly abundant and unavoidable in any *in vivo* setting, thereby interfering with any possible biosynthesis based on CoA-bound substrates. While the MAT crystal structure is solved (Rittner *et al.* 2020), it has not been envisioned that the crucial activating component of CoA may be replaced, let alone how an acyltransferase may be artificially reprogrammed so that it does not use Acyl-CoA substrates and, instead, specifically accepts subunits bound to different coenzyme moieties, such as synthetic coenzyme moieties.

Based on the crystal structure and the position of the CoA binding pocket therein (see **Fig. 1**), it was speculated that target residues that are highly attractive to reprogramming a natural acyltransferase could be identified. However, mutation experiments showed that amino acid exchanges at certain residues do not allow any functional protein expression. Therefore, an iterative testing strategy and protein engineering techniques combined with *in silico* searches were applied to define acyltransferase mutants that can be manipulated in a way to efficiently block CoA binding and -at the same time - that allow binding of different coenzyme moieties, while still allowing correct overall folding of the protein. Moreover, to offer a high versatility including the choice of acyltransferase protein itself, alignments of FAS amino acid sequences from various genera were performed in addition to the structural analysis. An alignment of 28 different example sequences is shown in **Table 1.** After extensive analysis, eight different target residues have been identified (see **Fig. 2**) in those sequences showing a certain degree of identity. To this end, various hot spots for mutational activities were defined that were tested in the following as further detailed below. To verify the identified hot sports in PKS ATs, four exemplary alignments were performed, grouped by the type of PKS ATs and using the FAS MAT as reference sequence. Correct alignment of the active site residues from the catalytic dyad (BB of a third residue involved) were used to judge the results.

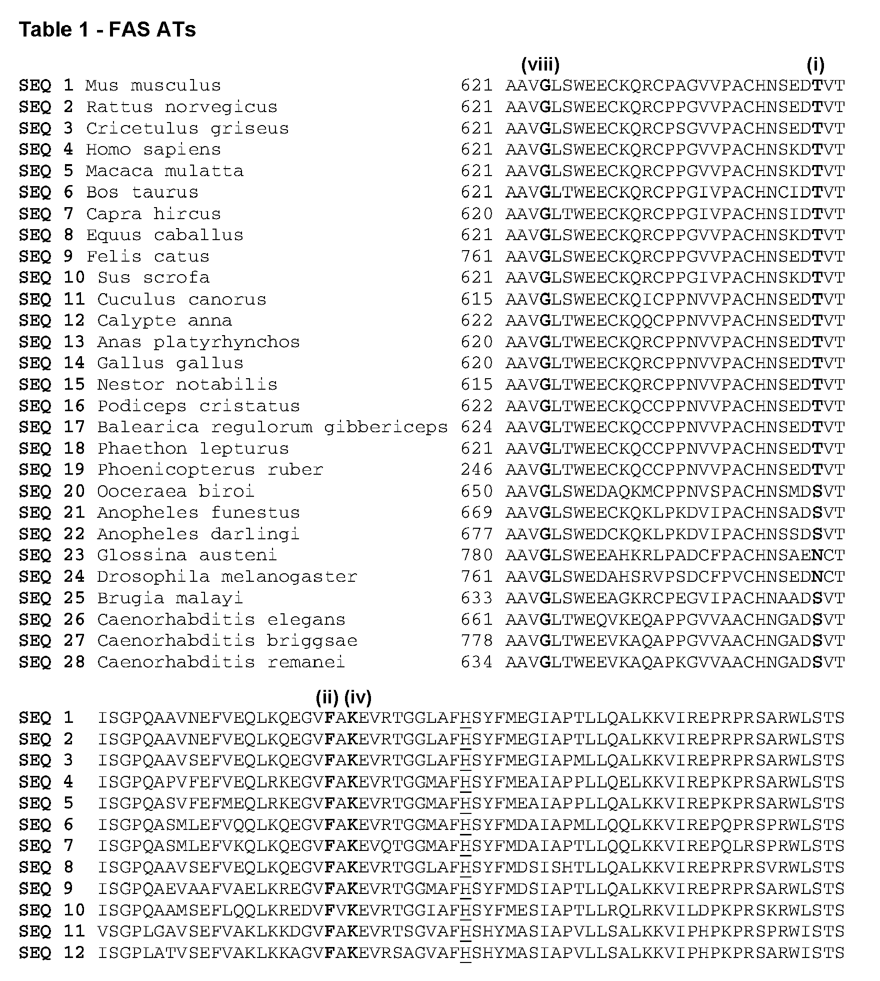

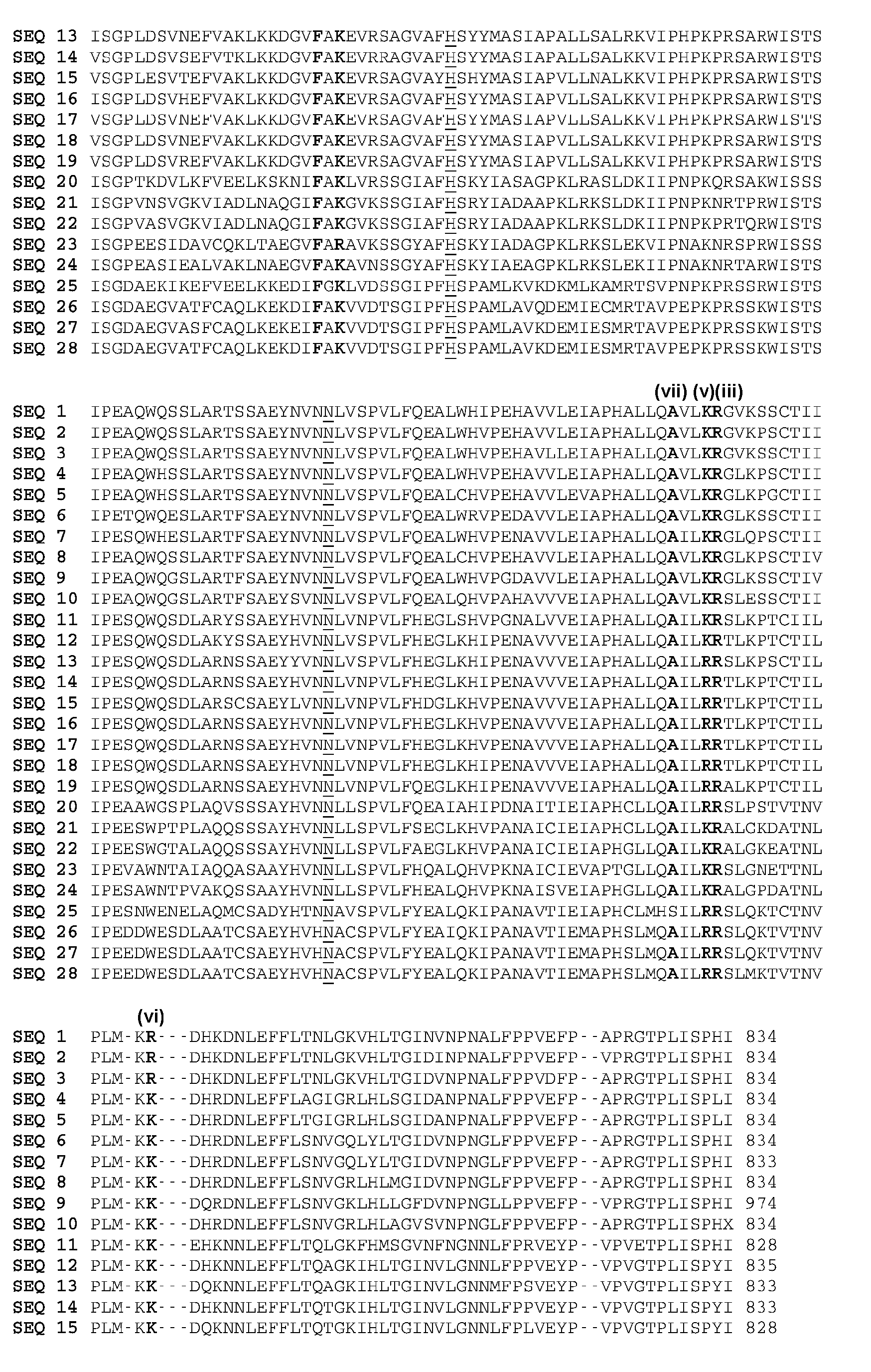

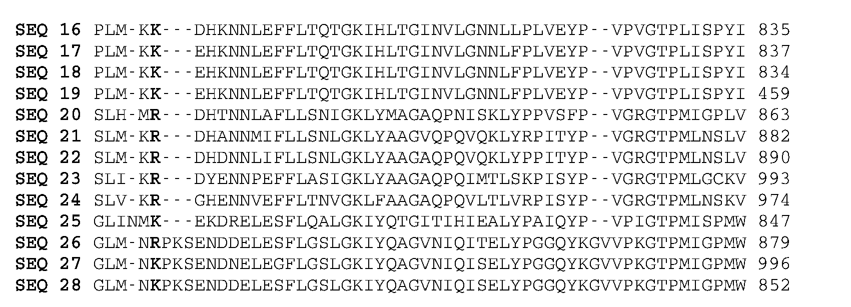

Catalytic positions are indicated as underlined, reference positions are indicated as bold with numbering of the reference positions shown on top.

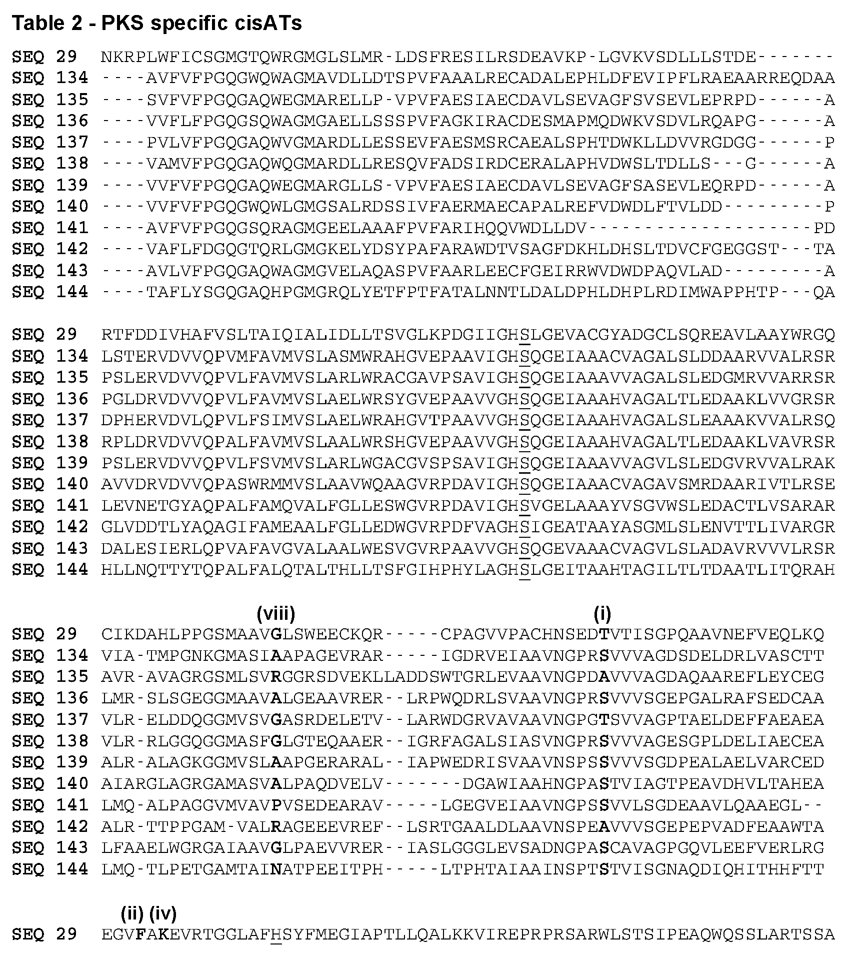

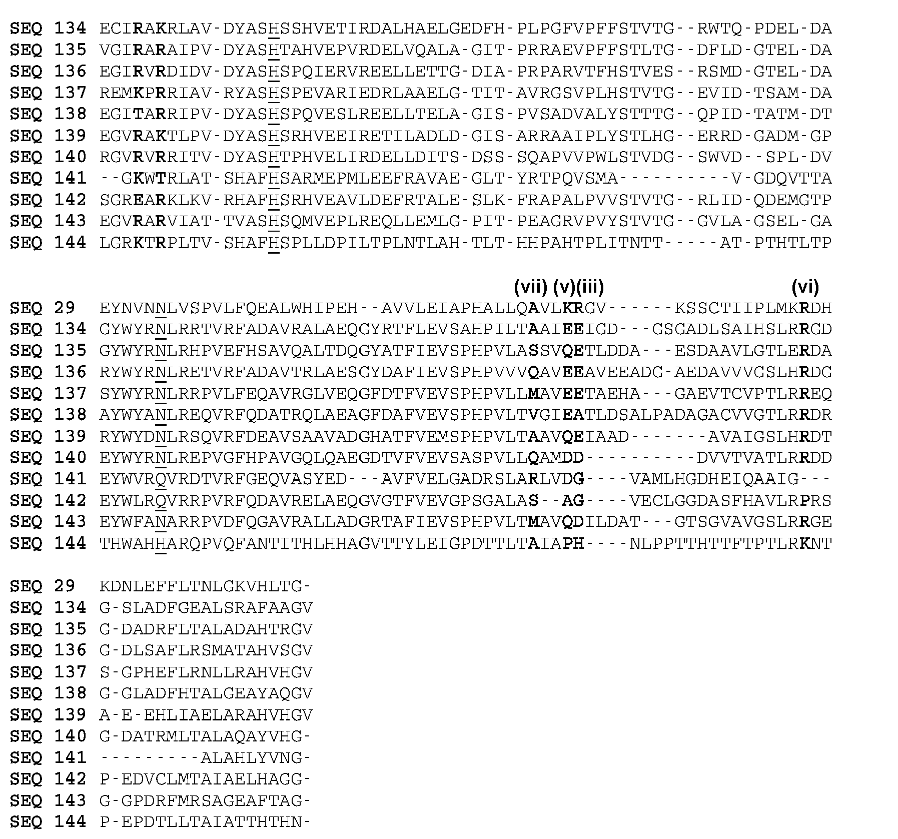

Catalytic positions are indicated as underlined, reference positions are indicated as bold with numbering of the reference positions shown on top. SEQ ID NO: 29: mouse MAT, SEQ ID NO: 134: DEBS1AT1, SEQ ID NO: 135: DEBS1AT2, SEQ ID NO: 136: DEBS2AT3, SEQ ID NO: 137: DEBS2AT4, SEQ ID NO: 138: DEBS3AT5, SEQ ID NO: 139: DEBS3AT6, SEQ ID NO: 140: RAPS3AT14, SEQ ID NO: 141: RAPS3AT12, SEQ ID NO: 142: RAPS2AT6, SEQ ID NO: 143: FluAAT1,SEQ ID NO: 144: FluAAT2.

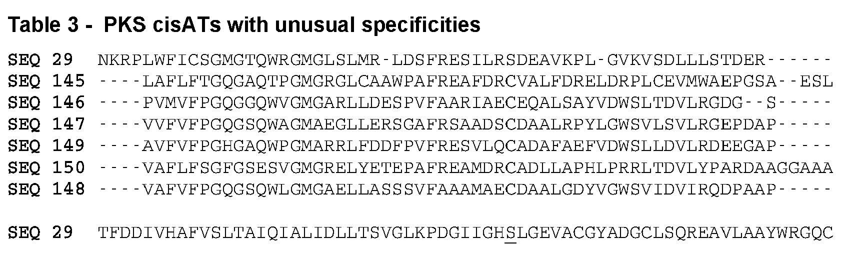

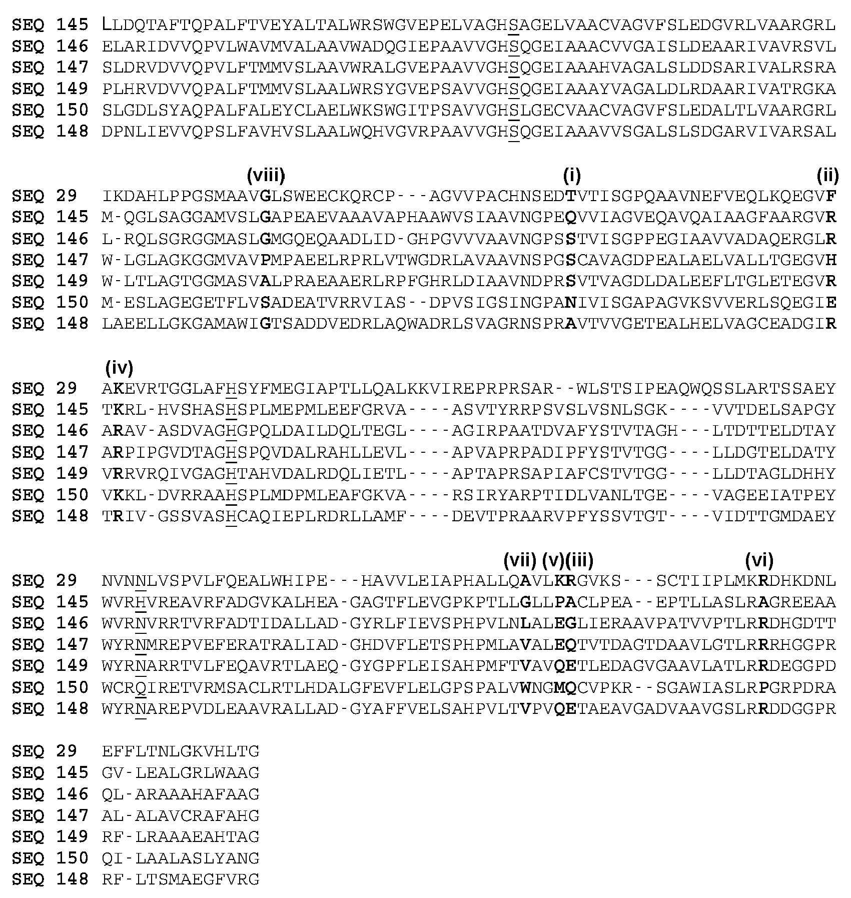

Catalytic positions are indicated as underlined, reference positions are indicated as bold with numbering of the reference positions shown on top. SEQ ID NO: 29: mouse MAT, SEQ ID NO: 145: EpoDAT4, SEQ ID NO: 146: MonAIVAT5, SEQ ID NO: 147: NidA3AT5, SEQ ID NO: 148: DivDAT6, SEQ ID NO: 149: SfalAT13 SEQ ID NO: 150: LeuAAT2.

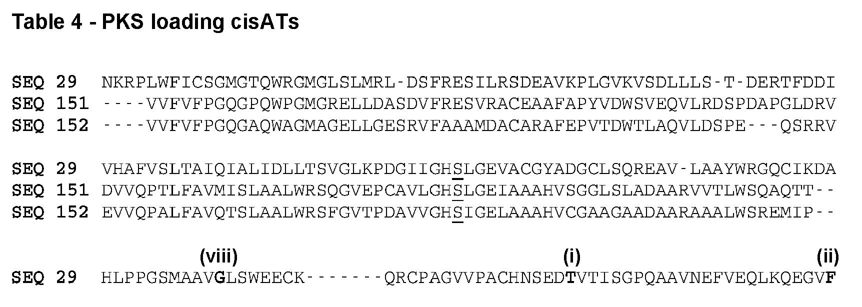

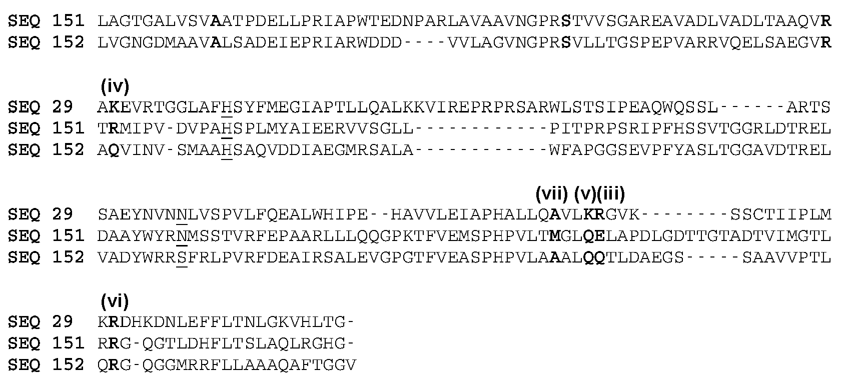

Catalytic positions are indicated as underlined, reference positions are indicated as bold with numbering of the reference positions shown on top. SEQ ID NO: 29: mouse MAT, SEQ ID NO: 151: AVES1AT0, SEQ ID NO: 152: DEBS1AT0.

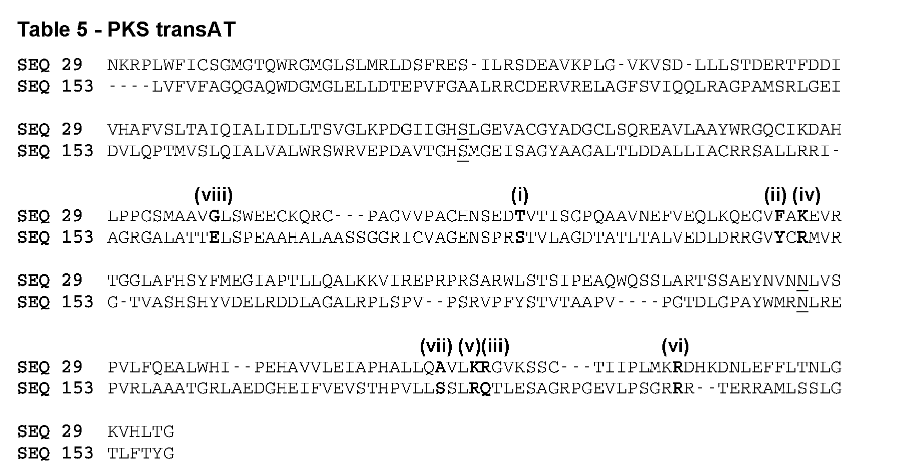

Catalytic positions are indicated as underlined, reference positions are indicated as bold with numbering of the reference positions shown on top. SEQ ID NO: 29: mouse MAT, SEQ ID NO: 153: KirCII.

### Example 2: Cloning and expression of altered acyltransferases

The basic plasmid encoding altered murine acyltransferases was prepared from a synthetic DNA fragment, containing a KS-LD-AT fragment with an inactive KS domain including Strep- and His-tags and was cloned into a pET22b vector using the restriction enzymes Ndel and Xhol, or with the in-Fusion Snap Assembly Master Mix (Takara). Mutations in the eight different positions corresponding to the reference positions according to the present invention were introduced by PCR using primers harboring the nucleotide variations. The plasmid encoding for the ACP was prepared similarly. An additional ribosome binding site (RBS) and another gene encoding for the 4'-phosphopantetheinyl transferase (PPT) Sfp was integrated into the same plasmid. The synthetic gene for the ACP was ordered with its native DNA sequence and codon optimized for *E. coli.*

The basic plasmid encoding altered DEBS module 6 acyltransferase (DEBS AT6) was prepared from genomic DNA of *Saccharopolyspora erythraea* (NRRL2338), containing a KS-LD-AT fragment with an inactive KS domain including His-tags and was cloned into a pET22b vector using the restriction enzymes Ndel and Nhel, or with the in-Fusion Snap Assembly Master Mix (Takara). Mutations in five different positions corresponding to the reference positions according to the present invention were introduced by PCR using primers harboring the nucleotide variations. The plasmid encoding for the ACP was prepared similarly. An additional ribosome binding site (RBS) and another gene encoding for the 4'-phosphopantetheinyl transferase superfamily protein from *Streptomyces platensis* was integrated into the same plasmid. The synthetic gene for the ACP and the PPT was ordered with its native DNA sequence and codon optimized for *E. coli.*

The PCR mixtures were treated with Dpn1 prior to DNA purification using electrophoresis. DNA was stained and illuminated with Sybr Green DNA Dye (Carl Roth). Appropriate fragments were isolated with a scalpel and DNA was purified with the GeneJet Gel Extraction kit (ThermoFisher). The fragments were transferred to Stellar^{™} Competent Cells (Takara) chemical competent cells for ligation and amplification of the targeted plasmids. The plasmids were isolated from cells using a standard commercial plasmid purification kit.

The plasmids encoding the MAT, DEBS AT6 or altered acyltransferases were expressed according to the literature (Rittner *et al.* 2018; Chen *et al.* 2006). Briefly, the plasmids were transformed into BL21(DE3) chemical competent cells (Agilent). Transformed cells were directly transferred to a pre-culture of 5 mL LB-medium containing ampicillin as a selection marker, which was grown over night at 37°C in an incubation shaker. The pre-culture was used to inoculate a 100-250 mL main-culture or 2 L culture (ACPs) in 2xYT or TB medium in a 500 mL or 5 L Erlenmeyer flask. The cultures were grown for 2 hours at 37°C and then the expression system was induced by adding 250 µM IPTG and were grown for another 16 hours at 20°C. The cells were lysed enzymatically in lysis buffer or centrifuged, re-suspended in lysis buffer containing DNasel and lysed by sonification (Hielscher UP200t, 43% amplitude, 5 minutes, on/off time 20 seconds). After centrifugation, the soluble fraction was purified by Ni-NTA (ThermoFisher) and Strep affinity chromatography (strep-tactin sepharose, IBA Lifesciences GmbH), if a Strep-tag was present, following the standard protocol. The DEBS AT6 variants and ACPs were purified by size-exclusion chromatography (ROTI Dex-25, carl-Roth) instead of Strep affinity chromatography as a second chromatographic purification. Purified proteins were frozen in aliquots in liquid nitrogen and stored at -80°C. Protein concentrations were determined by their absorbance at 280 nm. The absorbance was recorded on a NanoDrop One^{C} (ThermoFisher) and converted to concentration using the extinction coefficient of the respective protein, calculated from their primary sequence without the N-terminal methionine in Benchling.

### Example 3: Kinetic analysis of altered acyltransferases using MMal-CoA

An enzyme-coupled assay was performed to analyze the transfer of acyl-moieties from CoA to the ACP. Four solutions were prepared as 4x concentrated stocks in the assay buffer. Solution 1 contained the murine acyltransferase at the concentration range of 0.01 µM-10 µM and the acyltransferase DEBS AT6 at the concentration range of 0.2 µM-10 µM), depending on the transfer kinetics to achieve good signal to noise ratios. Solution 2 contained the enzyme-coupled system including 8 mM α-ketoglutaric acid, 1.6 mM NAD+, 1.6 mM TPP and 20 mU/100 µL αKGDH. Solution 3 contained the methylmalonyl (MMal)-CoA at concentrations between 0.1 µM and 1,000 µM or at a fixed concentration of 200 µM for the DEBS AT6 and solution 4 contained the ACP from murine FAS as a standalone protein at 240 µM or the ACP6 from DEBS as a standalone protein at 264 µM. 5 µL of Solutions 1-3 were pipetted into a 384-well Small Volume HiBase Microplates (Greiner Bio-one) and mixed manually. The reactions were started by adding solution 4 with the dispenser system of the microplate reader (ClarioStar, BMG labtech) or an electronic multichannel pipette (ThermoScientific, E1-ClipTip). The reaction progress was recorded using the following settings: 348-20 nm; emission: 476-20 nm; gain: 1500; focal height: 11.9 mm; flashes: 17; orbital averaging: off. Calibration was performed with reduced Nicotinamide adenine dinucleotide (NADH) under assay conditions.

Results of the methylmalonyl transfer of DEBS AT6 and variants thereof from MMal-CoA (50 µM) to ACP6 (66 µM) are shown in **Figure 5****.**

Kinetic data for exemplary FAS AT mutants is shown in Table 6.

**Table 6**

| **Mutant** | **SEQ ID** | **Mutation** | ***K*ₘ [µM]** | ***k*_{cat} [s⁻¹]** | ***k*_{cat/}*K*ₘ [M⁻¹ s⁻¹]** |
|---|---|---|---|---|---|
| (WT) | 29* | none | 0.62 | 4.000 | 6.5E+06 |
| (WT) | 29 | none | 0.53 | 1.297 | 2.5E+06 |
| A01 | 57 | T161A | 1.47 | 0.051 | 3.5E+04 |
| A02 | 58 | T161Y | 38.78 | 0.428 | 1.1E+04 |
| A03 | 59 | T161A F184A R286A | 3.41 | 0.021 | 6.2E+03 |
| A04 | 60 | T161A F184A K186A R286A | 7.24 | 0.013 | 1.8E+03 |
| A05 | 61 | K186E | 14.16 | 0.101 | 7.2E+03 |
| A06 | - | A282W | | Not determined | |
| A07 | 62 | K186D | 18.63 | 0.093 | 5.0E+03 |
| A08 | 63 | K285D | 0.11 | 0.133 | 1.2E+06 |
| A10 | 65 | R300D | 5.06 | 0.582 | 1.2E+05 |
| A11 | 66 | R300E | 3.33 | 0.834 | 2.5E+05 |
| A12 | 67 | K186D R300D | 66.23 | 0.019 | 2.9E+02 |
| A13 | 68 | K186D R300E | 685.98 | 0.190 | 2.8E+02 |
| A14 | 69 | K186E R300D | 218.27 | 0.051 | 2.3E+02 |
| A15 | 70 | K186E R300E | 58.39 | 0.030 | 5.2E+02 |

| | | | | | |
|---|---|---|---|---|---|
| Listed SEQ ID NOs refer to corresponding acyltransferase sequences. Mutations are indicated in reference to the wild type MAT sequence SEQ ID NO: 29. *Comparative data from Rittner *et al.* 2018 | | | | | |

### Example 4: Validation of altered acyltransferase activity

To verify the re-engineered mutant activity, having a substrate specificity altered from natural CoA substrates to non-CoA substrates, two alternative methods to quantify specific turnover rates for non-CoA substrates were established. Such non-CoA substrates are not detectable by the enzyme-coupled assay. We used a thiol-sensitive fluorophore (ThioGlo4; Yi *et al.* 2009) to quantify the released free thiol groups from the substrate *S*-Malonyl-*N-*hexanoylcysteamine or *S*-Methylmalonyl-*N*-hexanoylcysteamine after the enzyme-specific transfer of the malonyl moiety to a non-thiol acceptor or the methylmalonyl moiety to DEBS ACP6.

Briefly, three solutions were prepared in either a twofold concentration or as 4x concentrated stocks in the assay buffer. Solution 1 contained the murine acyltransferase variants at concentrations between 50-500 nM depending on their purified yields and were diluted twofold in the assay. Solution 2 contained a fixed concentration of the substrate *S-*Malonyl-*N*-hexanoylcysteamine at 200 µM and solution 3 contained the acceptor *N*-(2-hydroxyethyl)-hexanamide at 20 mM. 10 µL of Solutions 1 and 5 µL of Solution 2 were pipetted into a 384-well Small Volume HiBase Microplates (Greiner Bio-one). The reactions were started by adding 5 µL of solution 4 with a spichannel pipette (ClipTip) and incubated for 10 min at RT. The reactions were stopped by adding 2.5 µL 3 M HCl, neutralized by adding 2.5 µL 4 M NaOH and 10 µL of the reaction mixture was transferred to new wells. Free thiols were detected by adding 10 µL of 120 µM ThioGlo in the buffer 250 mM potassium phosphate, 1 mM EDTA and 10 % glycerol, mixing and fluorescence detection at the microplate reader (ClarioStar, BMG labtech) using the following settings: 400-20 nm; emission: 465-20 nm; gain: 700; focal height: 12.5 mm; flashes: 20; orbital averaging: off. Wells not containing any enzyme, but the substrate and acceptor in the mentioned concentrations served as the background and reflect non-enzyme specific transacylation.

Results of the malonyl transfer from the non-CoA substrate are shown in **Figure 4****.**

Alternatively, three solutions were prepared in either a twofold concentration or as 4x concentrated stocks in the assay buffer. Solution 1 contained the DEBS AT6 acyltransferase variants at concentrations between 4-10 µM depending on their purified yields and solution 2 contained the acceptor DEBS3 ACP6 at 266 µM. Both were diluted fourfold in the assay. Solution 3 contained a fixed concentration of the substrate *S-*Methylmalonyl-*N*-hexanoylcysteamine at 1000 µM and was diluted twofold in the assay. 12.5 µL of Solutions 1 and 12.5 µL of Solution 2 were pipetted into a 96-well plate using an electronic multichannel pipette (ThermoScientific, E1-ClipTip). The reactions were started by adding 25 µL of solution 3 with the electronic multichannel pipette and incubated for 10 min at RT. The reactions were stopped by adding 5 µL 1 M HCl and mixed with 2x 50 µL acetonitrile (ACN). 10 µL of the upper organic phases were transferred to a 384-well Small Volume HiBase Microplates (Greiner Bio-one) and the free thiols were detected by adding 15 µL of 120 µM ThioGlo in the buffer (250 mM potassium phosphate, 1 mM EDTA and 10 % glycerol), mixing, incubation for 5 min and fluorescence detection at the microplate reader (ClarioStar, BMG labtech) using the following settings: 400-20 nm; emission: 465-20 nm; gain: 700; focal height: 12.5 mm; flashes: 20; orbital averaging: off. Wells not containing any enzyme, but the substrate and acceptor in the mentioned concentrations served as the background and were subtracted from the measurements.

Results of the methylmalonyl transfer from the non-CoA substrate (500 µM)) to DEBS ACP6 (66 µM)) are shown in **Figure 5****.**

### Example 5: Chemobiosynthetic derivatization of 6-deoxyerythronolide B in a heterologous host

It is well established, that 6-deoxyerythronolide B (6-dEB) can be produced in specialized *Escherichia coli* (*E. coli*) cells when the three genes of the polyketide synthase DEBS (eryAI-eryAIII) and additional genes are heterologously expressed and a propionyl-thioester is fed to the culture (Pfeifer *et al.* 2001, Murli *et al.* 2005). This way, several 15-R-6-dEB analogues were produced by mutation of the loading module and feeding of different starter molecules as thioesters. The herein presented invention allows a general regioselective chemobiosynthetic derivatization of polyketides when the respective acyltransferase(s) are altered, and the respective function is fed as non-CoA thioester during cultivation.

Methods of the present invention are performed, for example, by production of 2-*R*-6-dEB analogues in specialized *Escherichia coli* cells by alteration of the DEBS AT6 and feeding of various elongation substrates during cultivation. For producing a specific derivative, e.g. 2-Fluoro-2-methyl-6-dEB, the native acyl specificity of the DEBS AT6 can be utilized and hence, for the reason of minimally engineering the polyketide synthase, it is only required to introduce point mutations into the DEBS AT6 as given in example 3 and to feed the culture with a 2-fluoro-2-methylmalonyl-thioester, linked via the thioester to a non-CoA coenzyme of the present invention. For producing a library of 2-*R*-6-dEB analogues, one would introduce the selectase by exchanging the native DEBS AT6, for example by exchanging the acyltransferase as in Rittner *et al.* (2022) and feed the culture(s) with various different elongation substrates as non-CoA substrates during cultivation.

Briefly, the encoding DNA of DEBS AT6 is exchanged with a DEBS AT6 variant DNA or a MAT variant DNA from example 2, as e.g. in Rittner *et al.* 2022, using the in-Fusion Snap Assembly Master Mix (Takara). The two plasmids encoding for DEBS1, DEBS2 and the altered DEBS3 together with additional genes are transformed into specialized (*E. coli*) cells, which are used to inoculate pre-cultures. These are used to inoculate production cultures, which are grown for 6-dEB production as described previously (Murli *et al.* 2003). Non-CoA substrates for chemobiosynthesis are added upon induction of the expression of T7 RNA polymerase at final concentrations around 4 mM unless otherwise stated. Sample broths for 6-dEB analysis were extracted with ethyl acetate and are analyzed by thin layer chromatography and staining with Vanilin or for deeper analysis with high-performance liquid chromatography (HPLC)/mass spectrometry (MS). For determination of the toxicity of non-CoA substrates a titration in the final concentration range between 0.5 mM-8 mM is performed (Murli *et al.* 2005).

### Example 6: In vivo polyketide derivatives production

Instead of manipulating the polyketide production in a heterologous host, the methods of the present invention can be used for producing polyketide derivatives directly in the native or industrial producing strain. This is achieved, for example, by production of 2-R-6-dEB analogues from *Saccharopolyspora erythraea* (*S. erythraea*) NRRL2338 variants with altered DEBS AT6 domains or swapped-in selectase domains, as described in example 5, and feeding of various non-CoA elongation substrates as thioesters during cultivation. The genetic manipulation of the producing actinomycetes strain can be accomplished by several well described methods, essentially by intergeneric conjugation with e.g. *E. coli* ET12567/pUZ8002 and subsequent induced homology-directed repair (HDR) (Tong *et al.* 2020).

The cultiviation of *S*. *erythraea* for production of erythromycin and derivatives thereof is well described in literature (Minas *et al.* 1998, Wu *et al.* 2011) and established protocols can be followed.

### References

Chen AY, Schnarr NA, Kim CY, Cane DE, Khosla C. Extender unit and acyl carrier protein specificity of ketosynthase domains of the 6-deoxyerythronolide B synthase. J Am Chem Soc. 2006 Mar 8;128(9):3067-74. doi: 10.1021/ja058093d.

Hertweck C. The biosynthetic logic of polyketide diversity. Angew Chem Int Ed Engl. 2009;48(26):4688-716. doi: 10.1002/anie.200806121.

Le, Q et al. Extraction of erythromycin from fermentation broth using salt-induced phase separation processes. Separation and Purification Technology,24 (1-2): 85-91, doi: 10.1016/S1383-5866(00)00217-3.

Minas W, Brünker P, Kallio PT, Bailey JE. Improved erythromycin production in a genetically engineered industrial strain of Saccharopolyspora erythraea. Biotechnol Prog. 1998 Jul-Aug;14(4):561-6. doi: 10.1021/bp980055t.

Murli S, Kennedy J, Dayem LC, Carney JR, Kealey JT. Metabolic engineering of Escherichia coli for improved 6-deoxyerythronolide B production. J Ind Microbiol Biotechnol. 2003 Aug;30(8):500-9. doi: 10.1007/s10295-003-0073-x.

Murli S, MacMillan KS, Hu Z, Ashley GW, Dong SD, Kealey JT, Reeves CD, Kennedy J. Chemobiosynthesis of novel 6-deoxyerythronolide B analogues by mutation of the loading module of 6-deoxyerythronolide B synthase 1. Appl Environ Microbiol. 2005 Aug;71(8):4503-9. doi: 10.1128/AEM.71.8.4503-4509.2005.

Pfeifer BA, Admiraal SJ, Gramajo H, Cane DE, Khosla C. Biosynthesis of complex polyketides in a metabolically engineered strain of E. coli. Science. 2001 Mar 2;291(5509):1790-2. doi: 10.1126/science.1058092.

Rittner A, Paithankar KS, Huu KV, Grininger M. Characterization of the Polyspecific Transferase of Murine Type I Fatty Acid Synthase (FAS) and Implications for Polyketide Synthase (PKS) Engineering. ACS Chem Biol. 2018 Mar 16;13(3):723-732. doi: 10.1021/acschembio.7b00718.

Rittner A, Paithankar KS, Himmler A, Grininger M. Type I fatty acid synthase trapped in the octanoyl-bound state. Protein Sci. 2020 Feb;29(2):589-605. doi: 10.1002/pro.3797.

Rittner, A., Joppe, M., Schmidt, J.J. et al. Chemoenzymatic synthesis of fluorinated polyketides. Nat. Chem. 14, 1000-1006 (2022). https://doi.org/10.1038/s41557-022-00996-z.

Smith S, Tsai SC. The type I fatty acid and polyketide synthases: a tale of two megasynthases. Nat Prod Rep. 2007 Oct;24(5):1041-72. doi: 10.1039/b603600g.

Tong Y, Whitford CM, Blin K, Jørgensen TS, Weber T, Lee SY. CRISPR-Cas9, CRISPRi and CRISPR-BEST-mediated genetic manipulation in streptomycetes. Nat Protoc. 2020 Aug;15(8):2470-2502. doi: 10.1038/s41596-020-0339-z.

Wu J, Zhang Q, Deng W, Qian J, Zhang S, Liu W. Toward improvement of erythromycin A production in an industrial Saccharopolyspora erythraea strain via facilitation of genetic manipulation with an artificial attB site for specific recombination. Appl Environ Microbiol. 2011 Nov;77(21):7508-16. doi: 10.1128/AEM.06034-11.

Yi L, Li H, Sun L, Liu L, Zhang C, Xi Z. A Highly Sensitive Fluorescence Probe for Fast Thiol-Quantification Assay of Glutathione Reductase. Angew. Chem. 2009; 121(22): 4094-4097. https://doi.org/10.1002/ange.200805693.

## Claims

1. A method comprising:
(a) providing
(i) at least one non-CoA acyltransferase substrate; and
(ii) at least one altered acyltransferase; and
(iii) at least one acyl carrier protein (ACP);
(b) allowing the at least one acyltransferase to transfer the acyl group of the at least one non-CoA acyltransferase substrate to the at least one ACP;
(c) obtaining at least one acyl-ACP;
wherein the at least one altered acyltransferase has a CoA-binding pocket that is altered to reduce CoA acyltransferase substrate transfer activity.

2. The method of claim 1, wherein step (b) is performed in at least one cell comprising the components (i), (ii), and (iii) of step (a).

3. The method of claim 1, wherein step (b) is performed outside of a cell, optionally, wherein the at least one altered acyltransferase is provided in a cell-free system and/or as a purified or partially purified protein or as a lysate comprising the altered acyltransferase, optionally wherein the at least one altered acyltransferase is provided as a fusion protein further comprising at least one linker domain and optionally at least one ketoacyl synthase domain.

4. A method for polyketide synthesis, the method comprising:
(a) providing
(i) at least one non-CoA acyltransferase substrate; and
(ii) optionally at least one CoA acyltransferase substrate; and
(iii) a polyketide synthase (PKS) system, wherein the PKS system comprises at least one altered acyltransferase;
(b) allowing polyketide synthesis, wherein the at least one non-CoA acyltransferase substrate, and optionally the at least one CoA acyltransferase substrate, is/are used as a substrate in the polyketide synthesis;
(c) obtaining at least one polyketide or derivative thereof, and optionally purifying said at least one polyketide or derivative thereof;
wherein the at least one altered acyltransferase has a CoA-binding pocket that is altered to reduce CoA acyltransferase substrate transfer activity.

5. The method of claim 4, wherein the PKS system is a PKS type I system or a PKS type II system, wherein one, two, three or more PKS modules comprise and/or interact with at least one altered acyltransferase.

6. The method of claim 4 or 5, wherein the first, second, third, fourth, fifth, six, seventh, eighth, ninth, tenth, eleventh, thirteenth, fourteenths, fifteenth, and/or sixteenth PKS module and/or one or more subsequent PKS modules comprise(s) and/or interact(s) with at least one altered acyltransferase.

7. The method of any one of claims 4 to 6, wherein PKS system is a PKS type I system and the at least one altered acyltransferase is part of at least one multifunctional PKS type I polypeptide, optionally through replacement of at least one of the PKS acyltransferases.

8. The method of any one of claims 4 to 7, wherein the at least one altered acyltransferase is provided as a separate polypeptide, optionally as a fusion protein further comprising at least one linker domain and optionally at least one ketoacyl synthase domain.

9. The method of any one of claims 4 to 8, wherein the polyketide synthesis, or part of the polyketide synthesis, is performed in at least one cell comprising the PKS system or parts thereof.

10. The method of any one of claims 4 to 9, wherein the polyketide synthesis, or part of the polyketide synthesis, is performed outside of a cell, optionally wherein the at least one altered acyltransferase is provided in a cell-free system and/or as a purified or partially purified protein or as a lysate comprising the altered acyltransferase, optionally wherein the at least one altered acyltransferase is provided as a fusion protein further comprising at least one linker domain and optionally at least one ketoacyl synthase domain.

11. The method of any of the previous claims, wherein the at least one altered acyltransferase has a CoA-binding pocket that is altered to transfer the at least one non-CoA acyltransferase substrate at a higher rate than the equivalent CoA acyltransferase substrate with the same acyl group and/or, in case at least one CoA acyltransferase substrate is provided in step (a), the at least one CoA acyltransferase substrate provided in step (a).

12. The method of any of the previous claims, wherein the at least one non-CoA substrate comprises or consists of
(ii) a non-CoA coenzyme; and
(i) an acyl group;
optionally wherein part (i) and part (ii) are linked via a thioester, optionally wherein
a) the non-CoA coenzyme has a neutral net charge or a positive net charge, further optionally , wherein the positive net charge is a single, two-fold, three-fold or four-fold positive net charge; and/or
b) wherein the non-CoA coenzyme does not comprise a negative charge.

13. The method of claim 12, wherein the acyl group comprises or consist of a malonyl group, an acetyl group, a methylmalonyl group, a propionyl group, an ethymalonyl group, or a derivative thereof.

14. Use of at least altered acyltransferase as defined in any one of claims 1 to 11, wherein the at least one altered acyltransferase originates from a PKS; and/or at least one non-CoA acyltransferase substrate as defined in claim 12 or 13, and optionally at least one CoA acyltransferase substrate for polyketide synthesis, fatty acid synthesis, and/or nonribosomal peptide synthesis.

15. A kit comprising at least altered acyltransferase as defined in any one of claims 1 to 11; and/or at least one non-CoA acyltransferase substrate as defined in claim 12 or 13; optionally further comprising at least one CoA acyltransferase substrate, and/or at least one container, and/or a set of reagents including buffer and/or medium, and/or means of transfection.
